# EUROPÄISCHE PATENTSCHRIFT

(11) **EP 0 680 949 B1**
(45) Veröffentlichungstag und Bekanntmachung des Hinweises auf die Patenterteilung: **08.04.1998**
(21) Anmeldenummer: 95106235.5
(22) Anmeldetag: 26.04.1995
(51) Int. Cl.: C07C 251/34, C07C 251/50, A01N 35/10, C07C 251/58

(54) **O-(Oxyimino)ethyl-Cyclohexenonoximether und deren Verwendung als Herbizide**
O-(oxyimino)ethyl-cyclohexenone oximethers and their use as herbicides
Ethers d'O-(oxyimino)éthyl-cyclohexenonoxime et leur utilisation comme herbicides

(30) Priorität: 05.05.1994 DE 4415871
(43) Veröffentlichungstag der Anmeldung: 08.11.1995
(73) Patentinhaber: BASF AKTIENGESELLSCHAFT, 67056 Ludwigshafen (DE)
(72) Erfinder: Misslitz, Ulf, Dr., D-67433 Neustadt (DE); Harreus, Albrecht, Dr., D-67063 Ludwigshafen (DE); König, Hartmann, Dr., D-69115 Heidelberg (DE); Walter, Helmut, Dr., D-67283 Obrigheim (DE); Westphalen, Karl-Otto, Dr., D-67346 Speyer (DE); Gerber, Matthias, Dr., D-67117 Limburgerhof (DE)

(56) Entgegenhaltungen:
- EP-A- 0 478 001
- WO-A-93/16063
- CHEMICAL ABSTRACTS, vol. 107, no. 17, 1987, Columbus, Ohio, US; abstract no. 154026c

## Beschreibung

Die vorliegende Erfindung betrifft neue O-(Oxyimino)ethyl-Cyclohexenonoximethern der Formel I 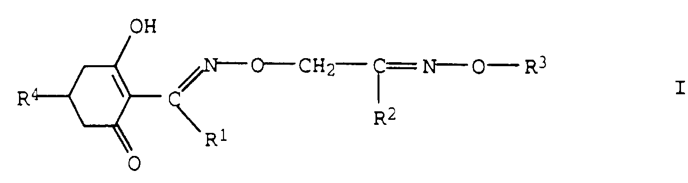 in der die Substituenten folgende Bedeutung haben:
- R¹: eine C₁-C₆-Alkylgruppe;
- R²: eine C₁-C₆-Alkylgruppe;
- R³: die Phenylgruppe, die unsubstituiert sein oder ein bis drei Substituenten tragen kann, ausgewählt aus der Gruppe bestehend aus Nitro, Cyano, Halogen, C₁-C₄-Alkyl und C₁-C₄-Halogenalkyl;
eine C₁-C₄-Alkyl-, C₃-C₄-Alkenyl- oder C₃-C₄-Alkinylgruppe, wobei diese Gruppen gewünschtenfalls einen der folgenden Substituenten tragen können:
Halogen, C₁-C₃-Alkyl,
Phenyl, das gewünschtenfalls seinerseits ein bis drei Reste tragen kann, ausgewählt aus der Gruppe bestehend aus Nitro, Cyano, Halogen, C₁-C₄-Alkyl, C₁-C₄-Halogenalkyl, Phenyl und Phenoxy, oder
Phenoxy, das gewünschtenfalls seinerseits ein bis drei Reste tragen kann, ausgewählt aus der Gruppe bestehend aus Nitro, Cyano, Halogen, C₁-C₄-Alkyl und C₁-C₄-Halogenalkyl;
- R⁴: eine C₁-C₄-Alkoxy-C₁-C₆-alkyl oder C₁-C₄-Alkylthio-C₁-C₆-alkylgruppe;
eine Phenylthio-C₁-C₆-alkylgruppe, wobei der Phenylring gewünschtenfalls ein bis drei Substituenten tragen kann, ausgewählt aus der Gruppe bestehend aus Halogen und C₁-C₄-Halogenalkyl;
eine N-(C₁-C₄-Alkylsulfonyl)-N-(C₁-C₄-alkyl)aminomethylgruppe;
eine C₃-C₇-Cycloalkyl- oder C₅-C₇-Cycloalkenylgruppe, wobei diese Gruppen unsubstituiert sein oder jeweils ein bis drei Substituenten tragen können, ausgewählt aus der Gruppe bestehend aus Hydroxyl, Halogen, C₁-C₄-Alkyl, C₁-C₄-Alkoxy, C₁-C₄-Alkylthio, C₁-C₄-Halogenalkyl;
ein 5-gliedriger gesättigter Heterocyclus, der ein oder zwei Sauerstoff- und/oder Schwefelatome als Heteroatome enthält und der unsubstituiert sein oder ein bis drei Substituenten tragen kann, ausgewählt aus der Gruppe bestehend aus C₁-C₄-Alkyl, C₁-C₄-Alkoxy, C₁-C₄-Alkylthio und C₁-C₄-Halogenalkyl;
ein 6- oder 7-gliedriger Heterocyclus mit einem oder zwei nicht benachbarten Sauerstoff- und/oder Schwefelatomen als Heteroatomen, der gesättigt oder ein- oder zweifach ungesättigt sein kann, wobei der Heterocyclus unsubstituiert sein oder ein bis drei Substituenten tragen kann, ausgewählt aus der Gruppe bestehend aus Hydroxyl, Halogen, C₁-C₄-Alkyl, C₁-C₄-Alkoxy, C₁-C₄-Alkylthio und C₁-C₄-Halogenalkyl;
ein 5-gliedriger Heteroaromat, enthaltend ein oder zwei Stickstoffatome und ein Sauerstoff- oder Schwefelatom, wobei der Heteroaromat unsubstituiert sein oder ein bis drei Substituenten tragen kann, ausgewählt aus der Gruppe bestehend aus Halogen, Cyano, C₁-C₄-Alkyl, C₁-C₄-Alkoxy, C₁-C₄-Alkylthio, C₁-C₄-Halogenalkyl, C₂-C₆-Alkenyl, C₂-C₆-Alkenyloxy und C₁-C₄-Alkoxy-C₁-C₄-alkyl;
die Phenyl- oder Pyridylgruppe, wobei diese Gruppen unsubstituiert sein oder jeweils ein bis drei Substituenten tragen können, ausgewählt aus der Gruppe bestehend aus Halogen, Nitro, Cyano, C₁-C₄-Alkyl, C₁-C₄-Alkoxy, C₁-C₄-Alkylthio, C₁-C₄-Halogenalkyl, C₃-C₆-Alkenyloxy, C₃-C₆-Alkinyloxy und -NR^{a}R^{b}, wobei
- R^{a}: für Wasserstoff, C₁-C₄-Alkyl, C₃-C₆-Alkenyl oder C₃-C₆-Alkinyl und
- R^{b}: für Wasserstoff, C₁-C₄-Alkyl, C₃-C₆-Alkenyl, C₃-C₆-Alkinyl, C₁-C₆-Acyl oder für Benzoyl steht, das gewünschtenfalls seinerseits ein bis drei Reste tragen kann, ausgewählt aus der Gruppe besehend aus Nitro, Cyano, Halogen, C₁-C₄-Alkyl, C₁-C₄-Alkoxy, C₁-C₄-Alkylthio und C₁-C₄-Halogenalkyl
stehen,
sowie die landwirtschaftlich brauchbaren Salze von I und die Ester von I mit C₁-C₁₀-Carbonsäuren oder anorganischen Säuren.

Außerdem betrifft die Erfindung die Verwendung dieser Verbindungen als Herbizide, herbizide Mittel, welche diese Verbindungen als wirksame Substanzen enthalten, Verfahren zur Herstellung dieser herbiziden Mittel sowie Verfahren zur Bekämpfung von unerwünschtem Pflanzenwuchs mit den Verbindungen I.

Des weiteren betrifft die Erfindung neue O-(Oxyimino)ethyl-Hydroxylamine der Formel III 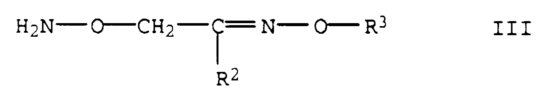 wobei
- R²: eine C₁-C₆-Alkylgruppe und
- R³: die Phenylgruppe, die unsubstituiert sein oder ein bis drei Substituenten tragen kann, ausgewählt aus der Gruppe bestehend aus Nitro, Cyano, Halogen, C₁-C₄-Alkyl und C₁-C₄-Halogenalkyl oder
eine C₁-C₄-Alkyl-, C₃-C₄-Alkenyl- oder C₃-C₄-Alkinylgruppe, wobei diese Gruppen gewünschtenfalls einen der folgenden Substituenten tragen können: Halogen, C₁-C₃-Alkyl, Phenyl, das unsubstituiert sein oder seinerseits ein bis drei Reste tragen kann, ausgewählt aus der Gruppe bestehend aus Nitro, Cyano, Halogen, C₁-C₄-Alkyl, C₁-C₄-Halogenalkyl, Phenyl und Phenoxy, oder Phenoxy, das gewünschtenfalls seinerseits ein bis drei Reste tragen kann, ausgewählt aus der Gruppe bestehend aus Nitro, Cyano, Halogen, C₁-C₄-Alkyl und C₁-C₄-Halogenalkyl,
bedeuten,
sowie die Ammonium-Salze der Verbindungen III mit Mineralsäuren.

Aus der Literatur sind bereits herbizid wirksame Cyclohexandione der Formel I' 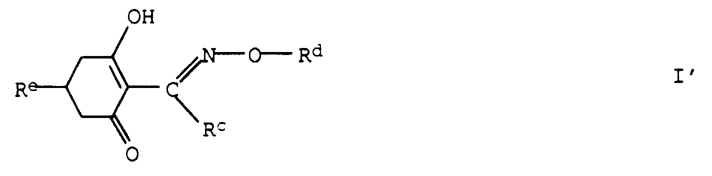 bekannt, wobei R^{c}, R^{d} und R^{e} u. a. für die folgenden Bedeutungen stehen:
- US 4,249,937 (R^{c} = Niederalkyl; R^{d} = Alkyl, Alkenyl; R^{e} = 2-(4-Chlorphenylthio)ethylen);
- WO 92/08696 (R^{c} = C1-C6-Alkyl; R^{d} = Benzyl; R^{e} = N-(Methylsulfonyl)-N-methyl-aminomethyl);
- WO 93/10081 (R^{c} = C₁-C₆-Alkyl; R^{d} = substituierter 3-Phenylpropenylenrest; R^{e} = C₁-C₆-Alkyl);
- US 4,440,566 (R^{c} = C₁-C₆-Alkyl; R^{d} = Benzyl; R^{e} = 2-Ethylthiopropyl);
- EP-A 238 021 und EP-A 125 094 (R^{c} = C₁-C₄- bzw. C₁-C₆-Alkyl; R^{d} = Benzyl, But-2-enyl; R^{e} = substituierter 5-gliedriger Heteroarylrest);
- EP-A 80 301 (R^{c} = C₁-C₆-Alkyl; R^{d} = Benzyl, But-2-enyl; R^{e} = substituiertes Phenyl);
- DE-A 38 38 309 (R^{c} = C₁-C₆-Alkyl; R^{d} = substituierter 4-Phenylbutylen- oder 4-Phenylbutenylenrest; R^{e} = substituierter 5- bis 7-gliedriger Heterocyclus);
- EP-A 456 112 (R^{c} = C₁-C₆-Alkyl; R^{d} = substituierter 3-Phenoxypropylen- oder 2-Phenoxyethylenrest; R^{e} = substituierter 5- bis 7-gliedriger Heterocyclus);
- WO-93/16,063 (R^{c} = C₁-C₆-Alkyl; R^{d} = substituierter (Benzylideniminooxy)alkylenrest; R^{e} = substituierter 5-7-gliedriger Heterocyclus);

Da die herbiziden Eigenschaften der bekannten Verbindungen, insbesondere hinsichtlich ihrer Selektivität gegen Ungraser in grasartigen Kulturpflanzen, nicht immer voll befriedigen, lagen der Erfindung neue Cyclohexenonoximether zugrunde, mit denen sich Ungräser in grasartigen Kulturen wie Reis und Mais besser als bisher gezielt bekämpfen lassen.

Demgemäß wurden die eingangs definierten O-(Oxyimino)ethyl-Cyclohexenonoximether I gefunden. Außerdem wurden ihre Verwendung als Herbizide, herbizide Mittel, die die Verbindungen I enthalten, ein Verfahren zur Herstellung dieser Mittel sowie ein Verfahren zur Bekämpfung von unerwünschtem Pflanzenwuchs mit den Verbindungen I gefunden.

Die O-(Oxyimino)ethyl-Cyclohexenonoximether I sind auf verschiedene Weise erhältlich, und zwar bevorzugt in an sich bekannter Weise aus schon bekannten Cyclohexenonen der Formel II (vgl. z.B. DE-A 38 38 309, EP-A 456 112, US 4,249,937 und WO 92/08696) und O-(Oxyimino)ethyl-Hydroxylaminen der Formel III: 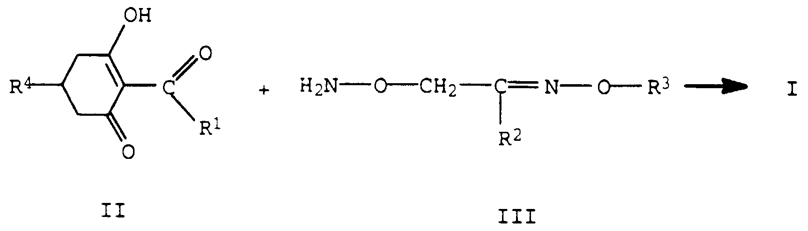 Vorzugsweise verwendet man ein geeignetes Salz des Hydroxylamins III, insbesondere dessen Hydrochlorid, und führt die Reaktion in heterogener Phase in einem inerten Lösungsmittel durch, beispielsweise in Dimethylsulfoxid, in einem Alkohol wie Methanol, Ethanol und Isopropanol, in einem aromatischen Kohlenwasserstoff wie Benzol und Toluol, in einem chlorierten Kohlenwasserstoff wie Chloroform und 1,2-Dichlorethan, in einem aliphatischen Kohlenwasserstoff wie Hexan und Cyclohexan, in einem Ester wie Essigsäureethylester oder in einem Ether wie Diethylether, Dioxan und Tetrahydrofuran.

Die Reaktionsführung erfolgt in Gegenwart einer Base, wobei normalerweise eine Basenmenge von etwa 0,5 bis 2 Mol-Äquivalent, bezogen auf die Ammoniumverbindung, ausreichend ist.

Als Basen kommen z. B. Carbonate, Hydrogencarbonate, Acetate, Alkoholate oder Oxide von Alkali- oder Erdalkalimetallen, insbesondere Natriumhydroxid, Kaliumhydroxid, Magnesiumoxid oder Calciumoxid in Betracht. Des weiteren sind organische Basen wie Pyridin und tert.-Amine wie Triethylamin geeignet.

Vorzugsweise führt man die Umsetzung in Methanol mit Natriumhydrogencarbonat als Base durch.

Eine Variante des Verfahrens besteht darin, die Umsetzung ohne Base mit der freien Hydroxylaminbase III, z. B. in Form einer wäßrigen Lösung, vorzunehmen; je nach verwendetem Lösungsmittel für die Verbindung II erhält man ein ein- oder zweiphasiges Reaktionsgemisch.

Geeignete Lösungsmittel für diese Variante sind beispielsweise Alkohole wie Methanol, Ethanol, Isopropanol und Cyclohexanol, aliphatische und aromatische, gegebenenfalls chlorierte Kohlenwasserstoffe wie Hexan, Cyclohexan, Methylenchlorid, Toluol und Dichlorethan, Ester wie Essigsäureethylester, Nitrile wie Acetonitril und cyclische Ether wie Dioxan und Tetrahydrofuran.

Zweckmäßigerweise setzt man das Cyclohexenon II und das O-(Oxyimino)ethyl-Hydroxylamin III bzw. dessen Salz in etwa stöchiometrischem Verhältnis ein, jedoch kann in manchen Fällen auch ein Überschuß der einen oder anderen Komponente, bis ca. 10 mol-%, vorteilhaft sein.

Die Reaktionstemperatur liegt im allgemeinen zwischen 0°C und dem Siedepunkt des Reaktionsgemisches, vorzugsweise bei 20 bis 80°C.

Die Reaktion ist nach wenigen Stunden beendet. Das Produkt kann auf übliche Weise, z. B. durch Einengen der Mischung, Verteilung des Rückstandes in Methylenchlorid/Wasser und Abdestillieren des Lösungsmittels unter vermindertem Druck, isoliert werden.

Besondere Bedingungen bezüglich des Drucks sind nicht zu beachten; im allgemeinen arbeitet man daher bei Normaldruck oder unter dem Eigendruck des jeweiligen Verdünnungsmittels.

Die erfindungsgemäßen Cyclohexenonoximether I können in Form ihrer landwirtschaftlich brauchbaren Salze oder als Enolester vorliegen, wobei es auf die Art des Salzes oder Esters im allgemeinen nicht ankommt. In der Regel sind zur Salzbildung solche Basen und zur Veresterung solche Säuren geeignet, welche die herbizide Wirkung von I nicht negativ beeinträchtigen.

Alkalimetallsalze der Verbindungen I können durch Behandeln der 3-Hydroxycyclohexenon-Verbindungen mit Natrium- oder Kaliumhydroxid bzw. -alkoholat in wäßriger Lösung oder in einem organischen Lösungsmittel wie Methanol, Ethanol, Aceton und Toluol erhalten werden.

Andere Metallsalze wie Mangan-, Kupfer-, Zink-, Eisen-, Calcium-, Magnesium- und Bariumsalze können aus den Natriumsalzen in üblicher Weise hergestellt werden, ebenso Ammonium-, Phosphonium-, Sulfonium- und Sulfoxoniumsalze mittels Ammonium-, Phosphonium-, Sulfonium- oder Sulfoxoniumhydroxiden.

Die Ester der Verbindungen I sind ebenfalls in üblicher Weise erhältlich (vgl. z. B. Organikum, VEB Deutscher Verlag der Wissenschaften, 17. Auflage, Berlin 1988, S. 405 - 408).

Die neuen O-(Oxyimino)ethyl-Hydroxylamine der Formel III lassen sich aus bekannten Vorprodukten über eine Reihe an sich bekannter Verfahrensschritte herstellen. Vorzugsweise koppelt man ein N-(2-Oxo-1-alkoxy)phthalimid IV {vgl. Pharmazie 25, 400 (1970)} auf für Ketone bekannte Weise (siehe hierzu z.B. Houben-Weyl, Methoden der organischen Chemie, Bd. E 14b, S. 369) unter Wasserabspaltung mit einem Hydroxylamin V (US 4,249,937, WO 92/08696, WO 93/10081, US 4,440,566, EP-A 238 021, EP-A 080 301, DE-A 38 38 309, EP-A 456 112, WO 93/16033, WO 93/1602): 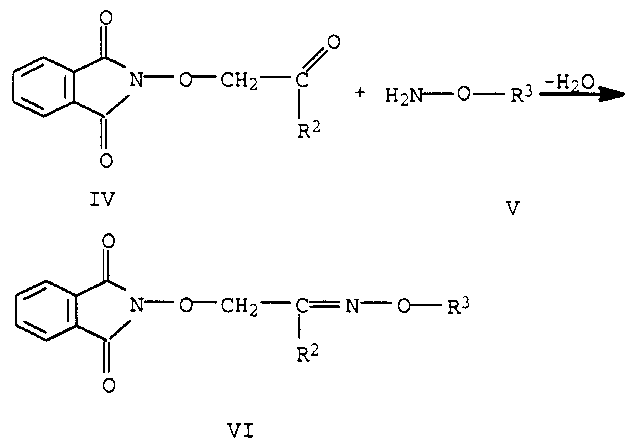 Die Oximetherderivate VI können aus den nach diesem Verfahren erhaltenen Reaktionsgemischen mittels üblicher Aufarbeitungsmethoden isoliert werden, beispielsweise durch Extraktion oder durch Kristallisation.

Vor ihrer Überführung in die O-(Oxyimino)ethyl-Hydroxylamine III können die Oximetherderivate VI gewünschtenfalls zwischengelagert werden.

Die Umwandlung in die O-(Oxyimino)ethyl-Hydroxylamine III (mit freier Aminogruppe) kann ebenfalls nach an sich bekannten Verfahren erfolgen. Hierzu sei insbesondere auf die Angaben in der DE-A 36 15 973 sowie in den darin zitierten Schriften verwiesen. Bevorzugt wird das Verfahren gemäß der DE-A 36 15 973 angewandt, nach dem die Hydroxylamine III mittels Ethanolamin freigesetzt wurden. Die Freisetzung der Hydroxylamine III mit Hilfe anderer Basen wie wäßrigen Mineralbasen, mit Aminen, Hydrazinen, Hydroxylaminen oder mittels wäßriger Säuren ist aber ebenfalls möglich: 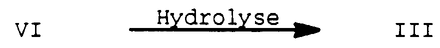 Die O-(Oxyimino)ethyl-Hydroxylamine III können aus den erhaltenen Reaktionsgemischen mittels üblicher Aufarbeitungsmethoden isoliert werden, beispielsweise durch Extraktion oder durch Kristallisation. Zur Erhöhung der Kristallisationstendenz kann es oftmals förderlich sein, die Hydroxylamine in ihre Salze mit Mineralsäuren oder organischen Säuren zu überführen. Dazu werden im allgemeinen verdünnte Lösungen der Säuren eingesetzt, wobei etwa stöchiometrische Mengen an Säure und Hydroxylamin-Derivat zweckmäßig sind.

Die erhaltenen Hydroxylammoniumsalze können wie die O-(Oxyimino)ethyl-Hydroxylamine III (mit freier Aminogruppe) direkt zu den Herbiziden der Formel I weiterverarbeitet werden oder auch, falls gewünscht, gelagert werden.

In Abhängigkeit von den Substituenten R² und R³ können die O-(Oxyimino)ethyl-Hydroxylamine III und die O-(Oxyimino)ethyl-Cyclohexenonoximether I bei der Herstellung als Isomerengemisch anfallen, wobei sowohl E-/Z-Isomerengemische als auch (R-/S-)Enantiomeren- oder Diastereoisomerengemische möglich sind. Die Isomerengemische können gewünschtenfalls nach den hierfür üblichen Methoden, z. B. durch Chromatographie oder durch Kristallisation, getrennt werden.

Die O-(Oxyimino)ethyl-Cyclohexenonoximether I können in mehreren tautomeren Formen geschrieben werden, die alle von der Erfindung umfaßt werden:

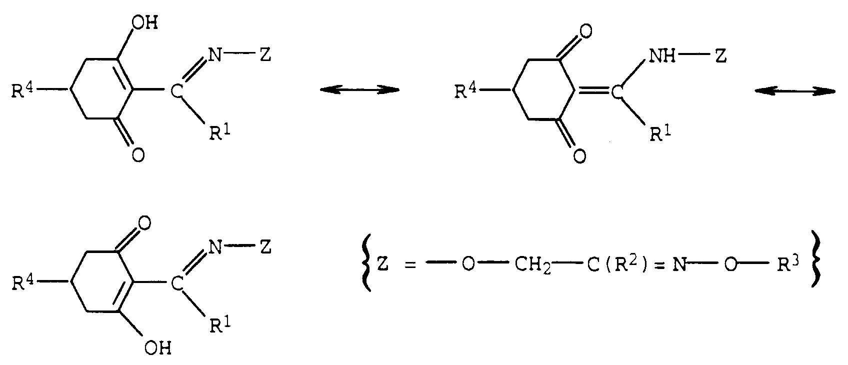

Die in den Definitionen der Substituenten verwendeten Sammelbegriffe
- Halogen,
- C₁-C₆-Alkylgruppe, C₁-C₄-Alkyl, C₁-C₃-Alkyl, C₁-C₄-Alkoxy, C₁-C₄-Alkylthio, C₁-C₄-Alkylsulfonyl, C₁-C₄-Halogenalkyl,
- C₃-C₇-Cycloalkylgruppe, C₅-C₇-Cycloalkenylgruppe,
- C₂-C₆-Alkenyl, C₃-C₆-Alkenyl, C₃-C₄-Alkenylgruppe, C₂-C₆-Alkenyloxy,
- C₃-C₆-Alkinyl, C₃-C₄-Alkinylgruppe, C₃-C₆-Alkinyloxy,
- N-(C₁-C₄-Alkylsulfonyl)-N-(C₁-C₄-alkyl)aminomethylgruppe,
- C₁-C₄-Alkoxy-C₁-C₄-alkyl, C₁-C₄-Alkoxy-C₁-C₆-alkylgruppe, C₁-C₄-Alkylthio-C₁-C₆-alkylgruppe,
- C₁-C₆-Acyl
stellen Kurzschreibweisen für eine individuelle Aufzählung der einzelnen Gruppenmitglieder dar. Sämtliche Alkyl-, Alkoxy-, Alkylthio-, Alkylsulfonyl, Halogenalkyl-, Alkenyl-, Alkenyloxy-, Alkinyl-, Alkinyloxy- und Acylteile können geradkettig oder verzweigt sein. Die Halogenalkylteile können gleich oder verschiedene Halogenatome tragen.

Im einzelnen bedeuten beispielsweise
- Halogen: Fluor, Chlor, Brom, Jod;
- C₁-C₆-Alkylgruppe: Methyl, Ethyl, n-Propyl, 1-Methylethyl, n-Butyl, 1-Methyl-propyl, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl;
- C₁-C₄-Alkyl(gruppe): Methyl, Ethyl, n-Propyl, 1-Methylethyl, n-Butyl, 1-Methylpropyl, 2-Methylpropyl, 1,1-Dimethylethyl;
- C₁-C₃-Alkyl: Methyl, Ethyl, n-Propyl, 1-Methylethyl;
- C₁-C₄-Alkoxy: Methoxy, Ethoxy, n-Propoxy, 1-Methyl-ethoxy, n-Butoxy, 1-Methylpropoxy, 2-Methylpropoxy, 1,1-Dimethylethoxy;
- C₁-C₄-Alkylthio: Methylthio, Ethylthio, n-Propylthio, 1-Methylethylthio, n-Butylthio, 1-Methylpropylthio, 2-Methylpropylthio, 1,1-Dimethylethylthio;
- C₁-C₄-Halogenalkyl: C₁-C₄-Alkyl wie vorstehend genannt, das partiell oder vollständig durch Fluor, Chlor und/oder Brom substituiert ist, also z.B, Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 3-Chlorpropyl, Heptafluorpropyl;
- C₂-C₆-Alkenyl: Ethenyl und C₃-C₆-Alkenyl wie 1-Propenyl, 2-Propenyl, 1-Methylethenyl, 1-Butenyl, 2-Butenyl, 3-Butenyl, 1-Methyl-1-propenyl, 1-Methyl-2-propenyl, 2-Methyl-1-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl;
- C₃-C₄-Alkenylgruppe: 1-Propenyl, 2-Propenyl, 1-Methylethenyl, 1-Butenyl, 2-Butenyl, 3-Butenyl, 1-Methyl-1-propenyl, 1-Methyl-2-propenyl, 2-Methyl-1-propenyl, 2-Methyl-2-propenyl;
- C₂-C₆-Alkenyloxy: Ethenyloxy und C₃-C₆-Alkenyloxy wie 2-Propenyloxy, 2-Butenyloxy, 3-Butenyloxy, 1-Methyl-2-propenyloxy, 2-Methyl-2-propenyloxy, 2-Pentenyloxy, 3-Pentenyloxy, 4-Pentenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-2-propenyloxy, 1-Ethyl-2-propenyloxy, 2-Hexenyloxy, 3-Hexenyloxy, 4-Hexenyloxy, 5-Hexenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 4-Methyl-4-pentenyloxy, 1,1-Dimethyl-2-butenyloxy, 1,2-Dimethyl-2-butenyloxy, 1,2-Dimethyl-3-butenyloxy, 1,3-Dimethyl-2-butenyloxy, 1,3-Dimethyl-3-butenyloxy, 2,2-Dimethyl-3-butenyloxy, 2,3-Dimethyl-2-butenyloxy, 2,3-Dimethyl-3-butenyloxy, 1-Ethyl-2-butenyloxy, 1-Ethyl-3-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy, 1-Ethyl-2-methyl-2-propenyloxy;
- C₃-C₆-Alkinyl: Prop-1-in-1-yl, Prop-2-in-3-yl, n-But-1-in-1-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-1-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-l-in-l-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl, 4-Methyl-pent-2-in-5-yl;
- C₃-C₄-Alkinylgruppe: Prop-1-in-1-yl, Prop-2-in-3-yl, n-But-1-in-1-yl, n-But-1-in-4-yl, n-But-2-in-1-yl;
- C₃-C₆-Alkinyloxy: Prop-1-in-1-yloxy, Prop-2-in-3-yloxy, n-But-1-in-1-yloxy, n-But-1-in-4-yloxy, n-But-2-in-1-yloxy, n-Pent-1-in-1-yloxy, n-Pent-1-in-3-yloxy, n-Pent-1-in-4-yloxy, n-Pent-1-in-5-yloxy, n-Pent-2-in-1-yloxy, n-Pent-2-in-4-yloxy, n-Pent-2-in-5-yloxy, 3-Methyl-but-1-in-1-yloxy, 3-Methyl-but-1-in-3-yloxy, 3-Methyl-but-1-in-4-yloxy, n-Hex-1-in-1-yloxy, n-Hex-1-in-3-yloxy, n-Hex-1-in-4-yloxy, n-Hex-1-in-5-yloxy, n-Hex-1-in-6-yloxy, n-Hex-2-in-1-yloxy, n-Hex-2-in-4-yloxy, n-Hex-2-in-5-yloxy, n-Hex-2-in-6-yloxy, n-Hex-3-in-1-yloxy, n-Hex-3-in-2-yloxy, 3-Methyl-pent-1-in-1-yloxy, 3-Methyl-pent-1-in-3-yloxy, 3-Methyl-pent-1-in-4-yloxy, 3-Methyl-pent-1-in-5-yloxy, 4-Methyl-pent-1-in-1-yloxy, 4-Methyl-pent-2-in-4-yloxy, 4-Methyl-pent-2-in-5-yloxy;
- C₃-C₇-Cycloalkylgruppe: Cyclopropyl, Cyclobutyl, Cyclopentyl, Cyclohexyl, Cycloheptyl;
- C₅-C₇-Cycloalkenyl: z.B. Cyclopent-1-enyl, Cyclopent-2-enyl, Cyclopent-3-enyl, Cyclohex-1-enyl, Cyclohex-2-enyl, Cyclohex-3-enyl, Cyclohept-1-enyl, Cyclohept-2-enyl, Cyclohept-3-enyl, Cyclohept-4-enyl.

Im Hinblick auf die herbizide Wirksamkeit der O-(Oxyimino)ethyl-Cyclohexenonoximether I sind die folgenden Bedeutungen der Substituenten, und zwar für sich allein oder in Kombination, besonders bevorzugt:
- R¹: Ethyl und Propyl;
- R²: Methyl;
- R³: die Phenylgruppe, unsubstituiert oder ein- bis dreifach substituiert durch
- Nitro, Cyano;
- Halogen, insbesondere Fluor, Chlor;
- C₁-C₄-Alkyl, insbesondere Methyl;
- C₁-C₄-Halogenalkyl, insbesondere Trifluormethyl;
eine C₁-C₄-Alkyl-, C₃-C₄-Alkenyl- oder C₃-C₄-Alkinylgruppe, wobei diese Gruppen gewünschtenfalls einen der folgenden Substituenten tragen können:
- Halogen, insbesondere Fluor, Chlor;
- C₁-C₃-Alkyl, insbesondere Methyl;
- Phenyl, das unsubstituiert sein oder ein bis drei Substituenten tragen kann, ausgewählt aus der Gruppe bestehend aus
   - Nitro, Cyano;
   - Halogen, insbesondere Fluor, Chlor;
   - C₁-C₄-Alkyl, insbesondere Methyl;
   - C₁-C₄-Halogenalkyl, insbesondere Trifluormethyl;
   - Phenyl, Phenoxy;
- Phenoxy, das unsubstituiert sein oder ein bis drei Substituenten tragen kann, ausgewählt aus der Gruppe bestehend aus
   - Nitro, Cyano;
   - Halogen, insbesondere Fluor, Chlor;
   - C₁-C₄-Alkyl, insbesondere Methyl;
   - C₁-C₄-Halogenalkyl, insbesondere Trifluormethyl;
- R⁴: eine C₁-C₆-Alkylgruppe wie vorstehend genannt, die durch C₁-C₄-Alkoxy, insbesondere Methoxy, Ethoxy, 1-Methylethoxy und 1,1-Dimethylethoxy, oder durch C₁-C₄-Alkylthio, insbesondere Methylthio und Ethylthio, substituiert ist, und zwar bevorzugt in 1-, 2- oder 3-Position;
ganz besonders bevorzugt ist 2-Ethylthiopropyl;
eine Phenylthio-C₁-C₆-alkylgruppe, insbesondere die 2-(Phenylthio)ethylgruppe, wobei der Phenylring gewünschtenfalls ein bis drei Halogenatome, insbesondere Fluor- und/oder Chloratome, und/oder C₁-C₄-Halogenalkylreste insbesondere Trifluormethyle, tragen kann;
eine N-(C₁-C₄-Alkylsulfonyl)-N-(C₁-C₄-alkyl)aminomethylgruppe; insbesondere die N-Methylsulfonyl-N-methylaminomethyl- oder N-Ethylsulfonyl-N-methyl-aminomethylgruppe;
eine C₃-C₇-Cycloalkyl- oder C₅-C₇-Cycloalkenylgruppe, die gewünschtenfalls ein bis drei der folgenden Reste tragen können: C₁-C₄-Alkyl, C₁-C₄-Alkoxy, C₁-C₄-Alkylthio und/oder C₁-C₄-Halogenalkyl; ganz besonders bevorzugt ist 1-Methylthio-1-cyclopropyl;
ein 5-gliedriger gesättigter Heterocyclus wie Tetrahydrofuranyl, Tetrahydrothienyl, Dioxolanyl, Dithiolanyl und Oxathiolanyl, insbesondere Tetrahydrofuranyl, Tetrahydrothienyl und Dioxolanyl, wobei der Heterocyclus unsubstituiert sein oder ein bis drei Reste tragen kann, ausgewählt aus der Gruppe bestehend aus C₁-C₄-Alkyl, C₁-C₄-Alkoxy, C₁-C₄-Alkylthio und C₁-C₄-Halogenalkyl;
ein 6- oder 7-gliedriger Heterocyclus der
a) gesättigt sein kann, z. B. Tetrahydropyranyl, Tetrahydrothiopyranyl, Oxepanyl, Thiepanyl und Dioxepan-5-yl,
b) ein- oder zweifach ungesättigt sein kann, z. B. Dihydropyran-3-yl, Dihydropyran-4-yl, Dihydrothiopyran-3-yl und Dihydrothiopyran-4-yl,
wobei die Heterocyclen unsubstituiert sein oder ein bis drei Reste tragen können, ausgewählt aus der Gruppe bestehend aus Hydroxyl, Halogen, C₁-C₄-Alkyl, C₁-C₄-Alkoxy, C₁-C₄-Alkylthio und C₁-C₄-Halogenalkyl;
ganz besonders bevorzugt sind Tetrahydropyran-3-yl, Tetrahydropyran-4-yl und Tetrahydrothiopyran-3-yl;
ein 5-gliedriger Heteroaromat wie Pyrrolyl, Pyrazolyl, Imidazolyl, Isoxazolyl, Oxazolyl, Isothiazolyl, Thiazolyl, Furanyl, Thienyl, 1,2,4-Oxadiazolyl, 1,2,4-Thiadiazolyl, 1,3,4-Oxadiazol-2-yl und 1,3,4-Thiadiazol-2-yl, vorzugsweise Isoxazolyl und Furanyl, wobei der Heteroaromat unsubstituiert sein oder ein bis drei Reste tragen kann, ausgewählt aus der Gruppe bestehend aus
- C₁-C₄-Alkyl, C₁-C₄-Alkoxy, C₁-C₄-Alkylthio, C₁-C₄-Halogenalkyl,
- C₁-C₄-Alkoxy-C₁-C₄-alkyl wie Methoxymethyl, 2-Methoxyethyl, 2-Methoxypropyl, 3-Methoxypropyl, 2-Methoxy--1-methylethyl, Ethoxymethyl, 2-Ethoxyethyl, 2-Ethoxypropyl, 3-Ethoxypropyl, 2-Ethoxy-1-methylethyl und 1-Ethoxy-1-methylethyl, insbesondere Methoxyethyl und Ethoxyethyl,
- C₂-C₆-Alkenyl wie Ethenyl und C₃-C₆-Alkenyl,
- C₂-C₆-Alkenyloxy, insbesondere 1-Methylethen-1-yloxy;
die Phenyl- oder Pyridylgruppe, die beide unsubstituiert sein oder insgesamt ein bis drei Reste tragen können, ausgewählt aus der Gruppe bestehend aus
- ein bis drei Resten C₁-C₄-Alkyl, C₁-C₄-Alkoxy, C₁-C₄-Alkylthio, C₁-C₄-Halogenalkyl, C₃-C₆-Alkenyloxy, C₃-C₆-Alkinyloxy, und
- einem Rest -NR^{a}R^{b}, wobei
   R^{a} für Wasserstoff,
   C₁-C₄-Alkyl, insbesondere Methyl und Ethyl, C₃-C₆-Alkenyl, insbesondere 2-Propenyl und 2-Butenyl, oder
   C₃-C₆-Alkinyl, insbesondere 2-Propinyl und 2-Butinyl,
   und
   R^{b} für Wasserstoff,
   C₁-C₄-Alkyl, insbesondere Methyl und Ethyl, C₃-C₆-Alkenyl, insbesondere 2-Propenyl und 2-Butenyl, C₃-C₆-Alkinyl, insbesondere 2-Propinyl und 2-Butinyl, C₁-C₆-Acyl wie Acetyl, Propionyl, n-Butyryl, 2-Methylpropionyl, Pentanoyl, 2-Methylbutyryl, 3-Methylbutyryl, 2,2-Dimethylpropionyl, n-Hexanoyl, 2-Methylpentanoyl, 2-Methylpentanoyl, 4-Methylpentanoyl, 2,2-Dimethylbutyryl, 2,3-Dimethylbutyryl, 3,3-Dimethylbutyryl und 2-Ethylbutyryl, insbesondere Acetyl und Propionyl,
   oder für
   Benzoyl, das seinerseits unsubstituiert sein oder ein bis drei Reste tragen kann, ausgewählt aus der Gruppe bestehend aus Nitro, Cyano, Halogen, vorzugsweise Fluor, Chlor und Brom, C₁-C₄-Alkyl, vorzugsweise Methyl, C₁-C₄-Alkoxy, vorzugsweise Methoxy und Ethoxy, C₁-C₄-Alkylthio, vorzugsweise Methylthio, sowie C₁-C₄-Halogenalkyl, vorzugsweise Trifluormethyl, stehen.

Als Salze der O-(Oxyimino)ethyl-Cyclohexenonoximether der Formel I kommen landwirtschaftlich brauchbare Salze, beispielsweise Alkalimetallsalze, insbesondere das Natrium- oder Kaliumsalz, Erdalkalimetallsalze, insbesondere das Calcium-, Magnesium- oder Bariumsalz, Mangan-, Kupfer-, Zink- oder Eisensalz sowie Ammonium-, Phosphonium-, Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoxoniumsalze in Betracht.

Unter landwirtschaftlich brauchbaren Estern sind vorzugsweise die Ester von C₁-C₁₀-Fettsäuren, insbesondere C₁-C₆-Alkylcarbonsäuren wie Methylcarbonsäure (Essigsäure), Ethylcarbonsäure (Propionsäure), Propylcarbonsäure (Buttersäure), 1-Methylethylcarbonsäure (Isobuttersäure), Butylcarbonsäure, 1-Methylpropylcarbonsäure, 2-Methylpropylcarbonsäure, 1,1-Dimethylethylcarbonsäure, Pentylcarbonsäure, 1-Methylbutylcarbonsäure, 2-Methylbutylcarbonsäure, 3-Methylbutylcarbonsäure, 1,1-Dimethylpropylcarbonsäure, 1,2-Dimethylpropylcarbonsäure, 2,2-Dimethylpropylcarbonsäure, 1-Ethylpropylcarbonsäure, Benzoesäure sowie durch Halogen substituierte Benzoesäuren, Hexylcarbonsäure, 1-Methylpentylcarbonsäure, 2-Methylpentylcarbonsäure, 3-Methylpentylcarbonsaure, 4-Methylpentylcarbonsäure, 1,1-Dimethylbutylcarbonsäure, 1,2-Dimethylbutylcarbonsäure, 1,3-Dimethylbutylcarbonsäure, 2,2-Dimethylbutylcarbonsäure, 2,3-Dimethylbutylcarbonsäure, 3,3-Dimethyl-butylcarbonsäure, 1-Ethylbutylcarbonsäure, 2-Ethylbutylcarbonsäure, 1,1,2-Trimethylpropylcarbonsäure, 1,2,2-Trimethylpropylcarbonsäure, 1-Ethyl-1-methyl-propylcarbonsäure und 1-Ethyl-2-methylpropylcarbonsäure, zu verstehen.

Die O-(Oxyimino)ethyl-Cyclohexenonoximether I, deren Salze und Ester, eignen sich - sowohl als Isomerengemische als auch in Form der reinen Isomeren - als Herbizide, insbesondere zur Bekämpfung von Pflanzenarten aus der Familie der Gräser (Gramineen). Im allgemeinen sind sie verträglich und somit selektiv in breitblättrigen Kulturen sowie in monokotylen (einkeimblättrigen) Gewächsen, welche nicht zu den Gramineen zählen. Einige der erfindungsgemäßen Verbindungen I sind auch zur selektiven Bekämpfung von unerwünschten Gräsern in Gramineenkulturen geeignet.

Die selektive Wirkung gegen Unkräuter und Schadgräser in Kulturen wie Weizen, Reis, Mais, Soja und Baumwolle tritt vor allem bei niedrigen Aufwandmengen auf.

Die O-(Oxyimino)ethyl-Cyclohexenonoximether I, deren Salze und Ester, bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Methanol, Ethanol, Propanol, Butanol, Cyclohexanol, Cyclohexanon oder stark polare Lösungsmittel, wie N-Methylpyrrolidon oder Wasser in Betracht.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylen-alkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Formulierungen enthalten im allgemeinen zwischen 0,01 und 95 Gew. %, vorzugsweise zwischen 0,5 und 90 Gew. %, mindestens einer Verbindung I. Die Wirkstoffe werden dabei in einer Reinheit von 90% bis 100%, vorzugsweise 95% bis 100% (nach NMR-Spektrum) eingesetzt.

Die erfindungsgemäßen Verbindungen I können beispielsweise wie folgt formuliert werden:
I. 20 Gewichtsteile der Verbindung Nr. 3.05 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.
II. 20 Gewichtsteile der Verbindung Nr. 4.01 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
III.20 Gewichtsteile des Wirkstoffs Nr. 4.03 werden in einer Mischung gelöst, die aus 25 Gewichtsteilen Cyclohexanon, 65 Gewichtsteilen einer Mineralölfraktion vom Siedepunkt 210 bis 280 ⁰C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.
IV. 20 Gewichtsteile des Wirkstoffs Nr. 15.09 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.
V. 3 Gewichtsteile des Wirkstoffs Nr. 9.04 werden mit 97 Gewichtsteilen feinteiligem Kaolin vermischt. Man erhält auf diese Weise ein Stäubemittel, das 3 Gew.% des Wirkstoffs enthält.
VI. 20 Gewichtsteile des Wirkstoffs Nr. 12.01 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1,0 kg/ha mindestens einer aktiven Substanz (a.S.) der Formel I.

In Anbetracht der Vielseitigkeit der Applikationsmethoden können die O-(Oxyimino)ethyl-Cyclohexenonoximether I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N.rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (S. vulgare), Theobroma cacao, Trifolium pratense, Triticum aestivum, Triticum durum, Vicia faba, Vitis vinifera, Zea mays.

Zur Verbreiterung des Wirkungsspektrums und zur Erzielung synergistischer Effekte können die O-(Oxyimino)ethyl-Cyclohexenonoximether I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Herstellungsbeispiel

### 2-[1-{2-[2-(4-Chlorphenoxy)ethoxyimino]propoxyimino}butyl]-3-hydroxy-5-(2H-tetrahydrothiopyran-3-yl)-2-cyclohexen-1-on (Verbindung Nr. 3.04)

Eine Mischung aus 1,00 g (3,56 mmol) 3-Hydroxy-2-butyryl-5-(2H-tetrahydrothiopyran-3-yl)-2-cyclohexen-1-on, 0,92 g (3,56 mmol) O-{2-[2-(4-Chlorphenoxy)ethoxyimino]propyl}hydroxylamin und 100 ml Methanol wurde 16 Std. bei 25°C gerührt. Anschließend engte man das Reaktionsgemisch unter reduziertem Druck ein, wonach der Rückstand in bekannter Weise auf das Produkt hin aufgearbeitet wurde. Ausbeute: 65 %
- ¹H-NMR(400 MHZ, in CDCl₃):: δ [ppm] = 4,20 (m,2H), 4,40 (m,2H), 6,85 (m,2H), 7,20 (m,2H) *)

*) ausgewählte, repräsentative Signale

### Vorstufe:

### O-{2-[2-(4-Chlorphenoxy)ethoxyimino]propyl}hydroxylamin

Eine Mischung aus 5,30 g (24 mmol) N-(2-Oxo-1-propoxy)phthalimid {vgl. Pharmazie 25, 400 (1970)}, 4,50 g (24 mmol) o-[2-(4-Chlorphenoxy)ethyl]hydroxylamin (vgl. EP-A 456,112) und 120 ml Methanol wurde 16 Std. bei 25°C gerührt, wonach man bei reduziertem Druck einengte. Das verbleibende Öl (10 g) wurde mit 100 ml Ethanolamin versetzt. Nach 4 Std. Rühren bei 40°C rührte man die Mischung in Wasser ein und extrahierte dann dreimal mit Methylenchlorid. Die vereinigten organischen Phasen wurden mit Wasser gewaschen, über Natriumsulfat getrocknet und eingeengt. Ausbeute: 79 %
¹H-NMR (200 MHZ, in CDCl₃):
- Hauptisomer(rel. Anteil: 80 %):: δ [ppm] = 1,35 (d,3H), 1,80 (s,3H), 4,00-4,30 (m,2H), 4,15 (s,2H), 4,65 (m,1H), 5,45 (bs,2H), 6,85 (m,2H), 7,20 (m,2H).
- Nebenisomer(rel. Anteil: 20 %):: δ [ppm] = 1,30 (d,3H), 1,95 (s,3H), 4,00-4,30 (m,2H), 4,15 (s,2H), 4,65 (m,1H), 5,45 (bs,2H), 6,85 (m,2H), 7,20 (m,2H).

In der folgenden Tabelle 1 sind weitere O-(Oxyimino)ethyl-Hydroxylamine III aufgeführt, die auf die gleiche Weise hergestellt wurden oder herstellbar sind. Die Tabellen 2 bis 24 enthalten erfindungsgemäße O-(Oxyimino)ethyl-Cyclohexenonoximether I⁻.

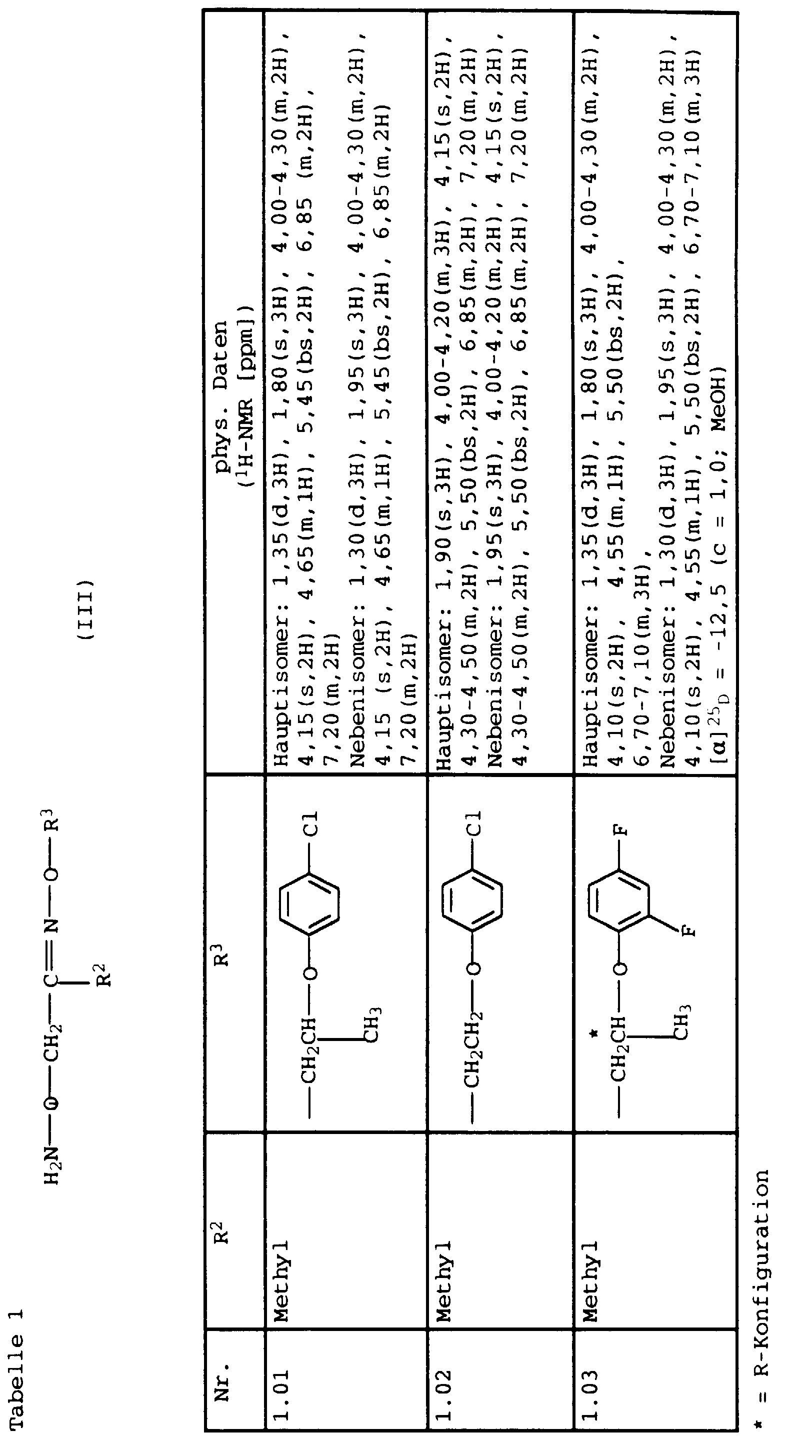

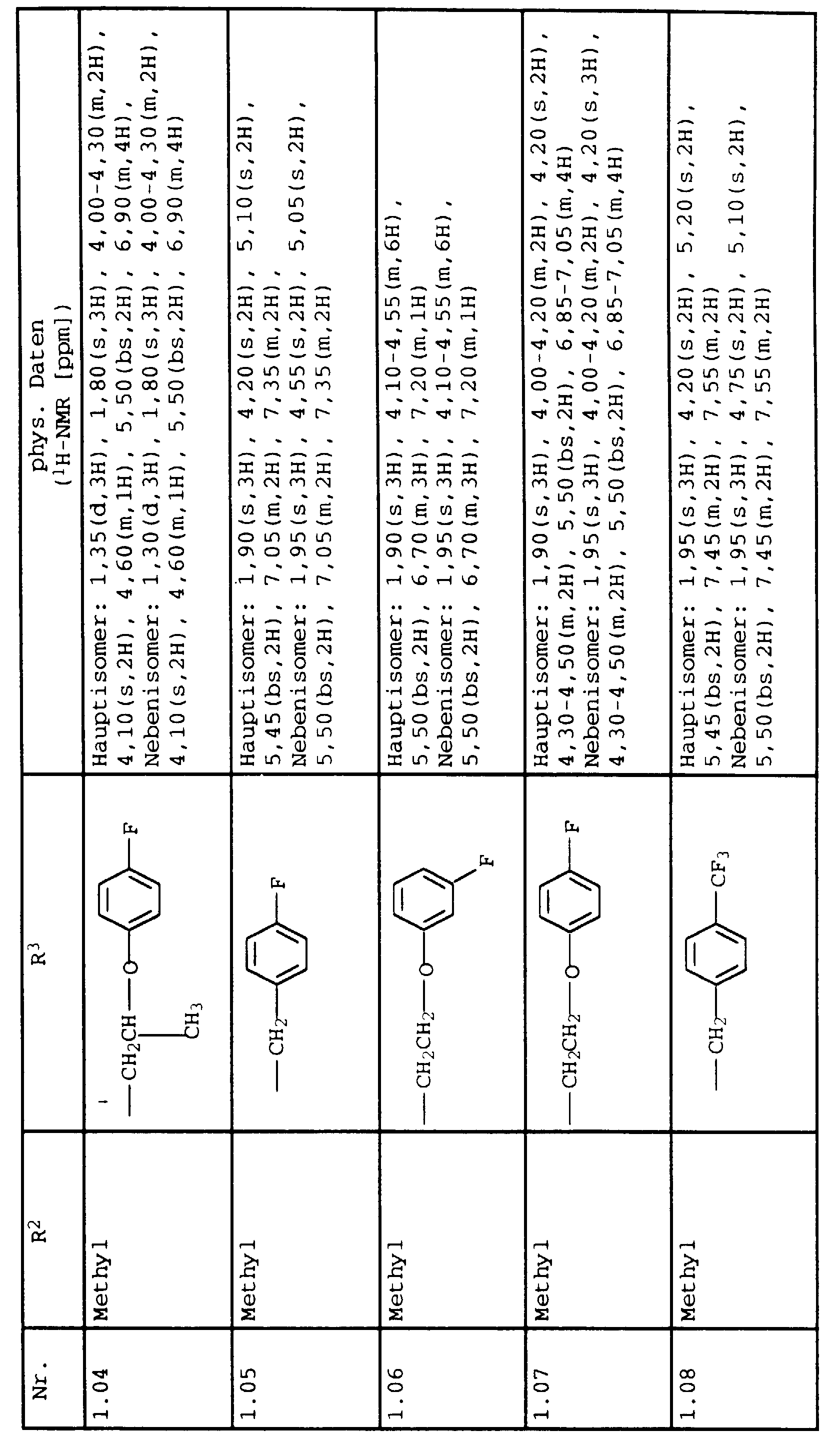

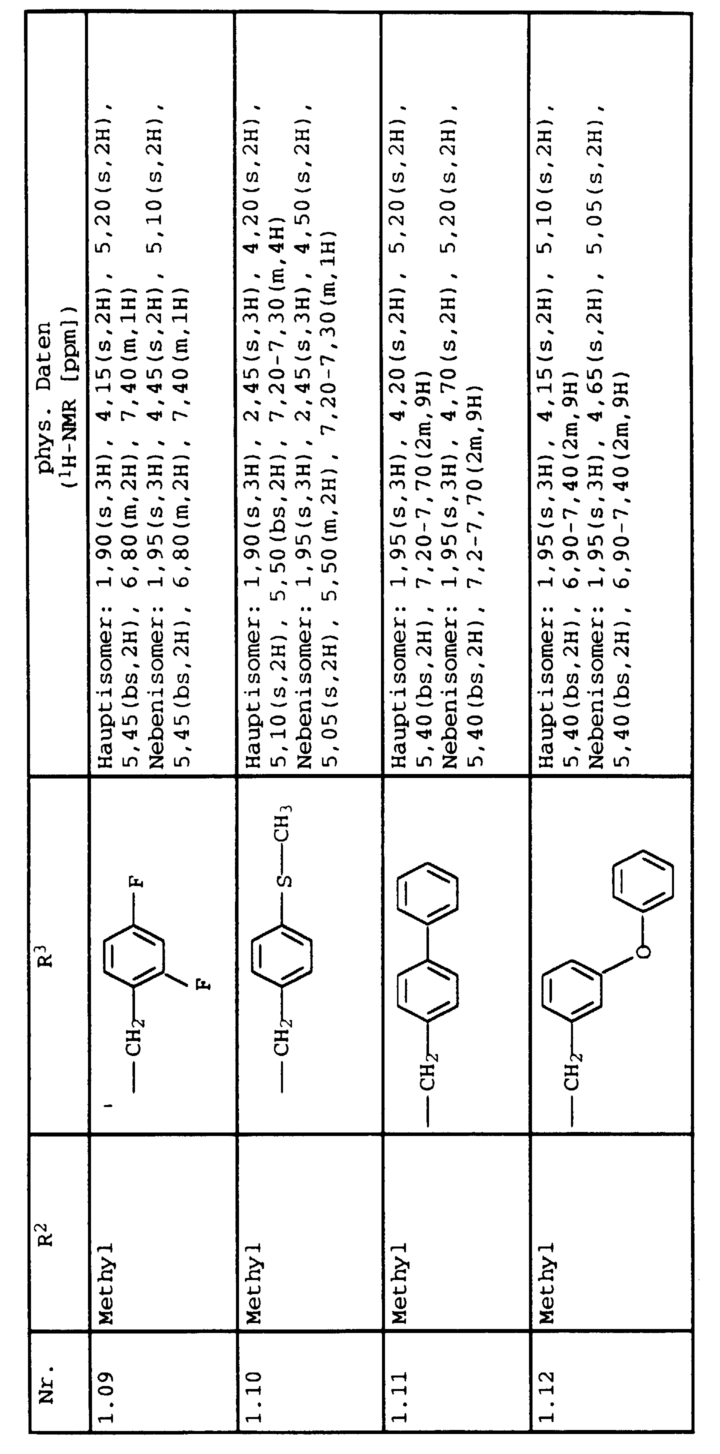

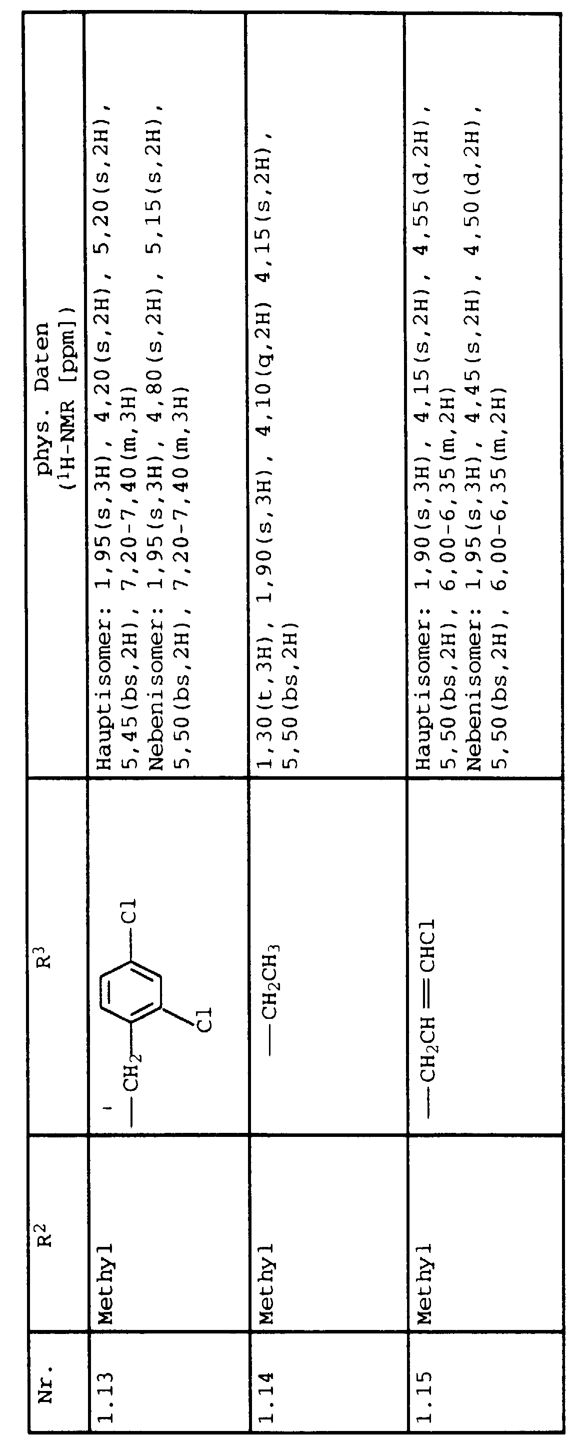

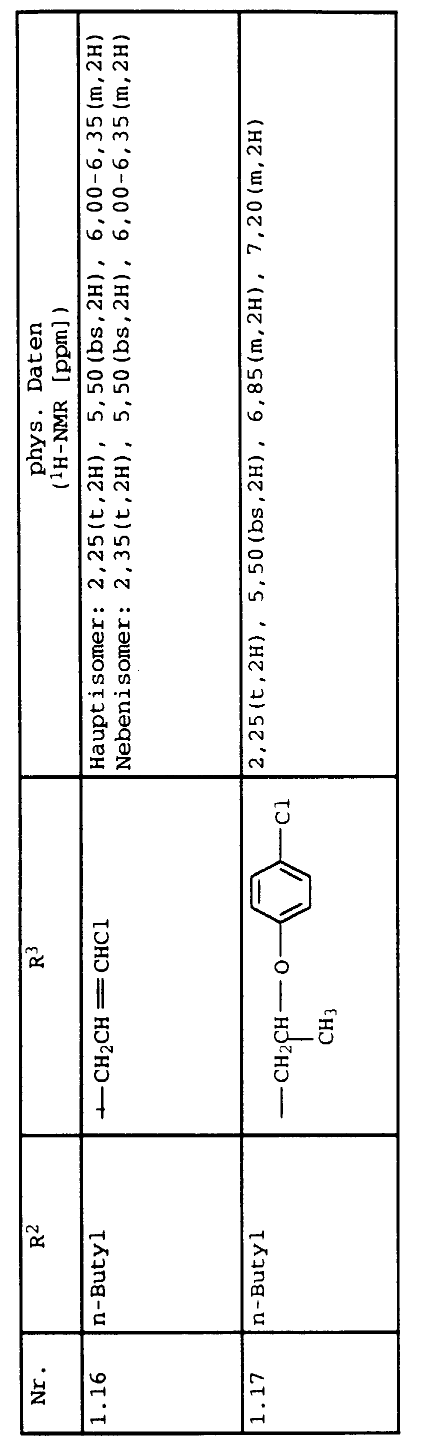

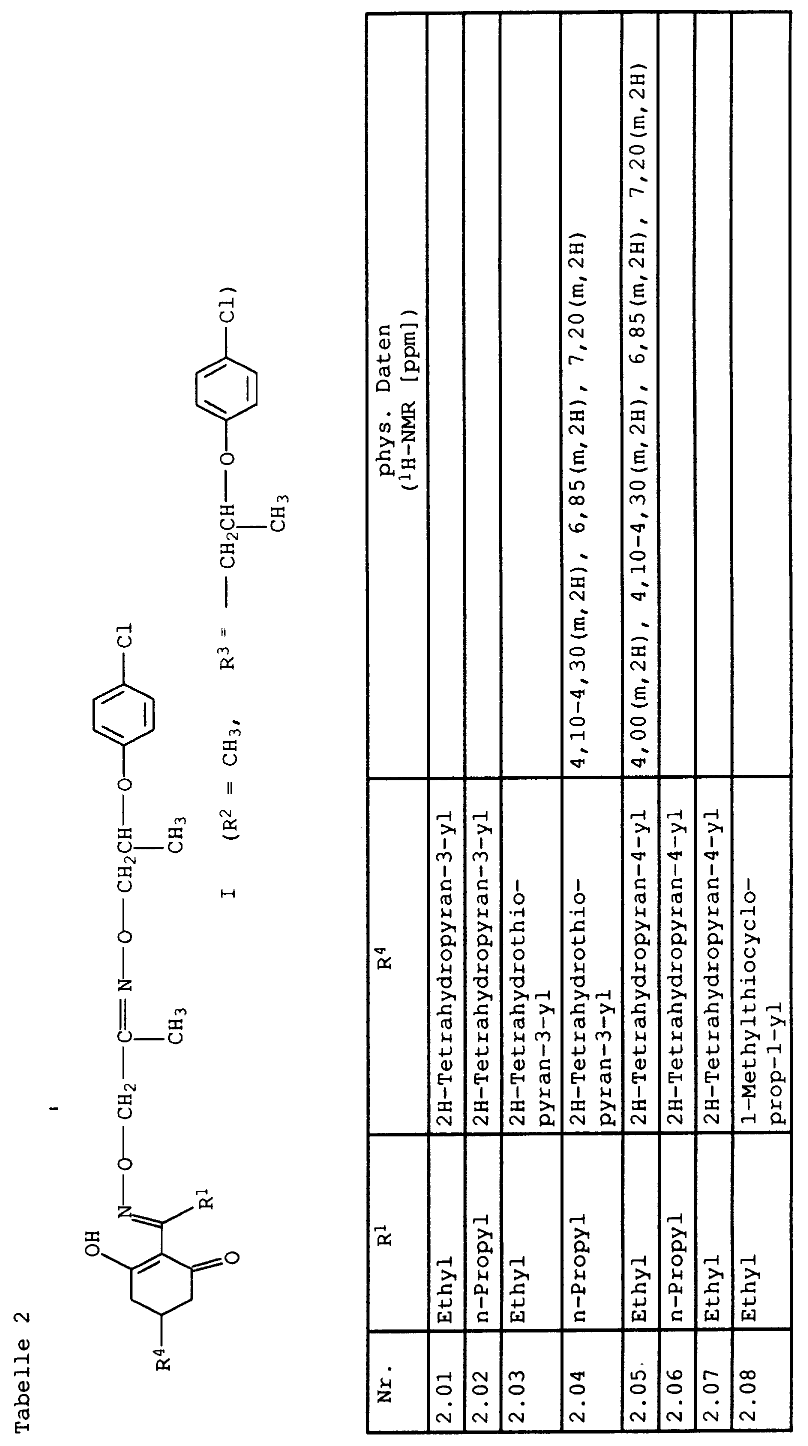

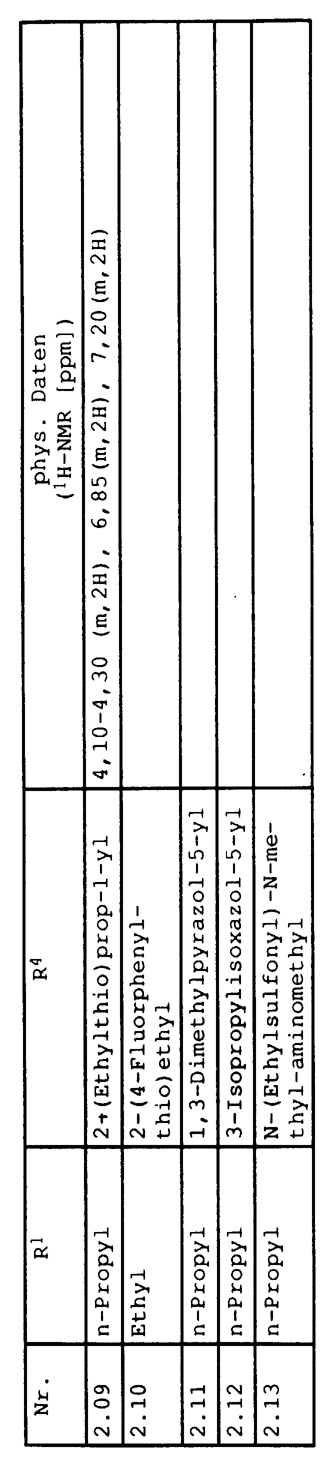

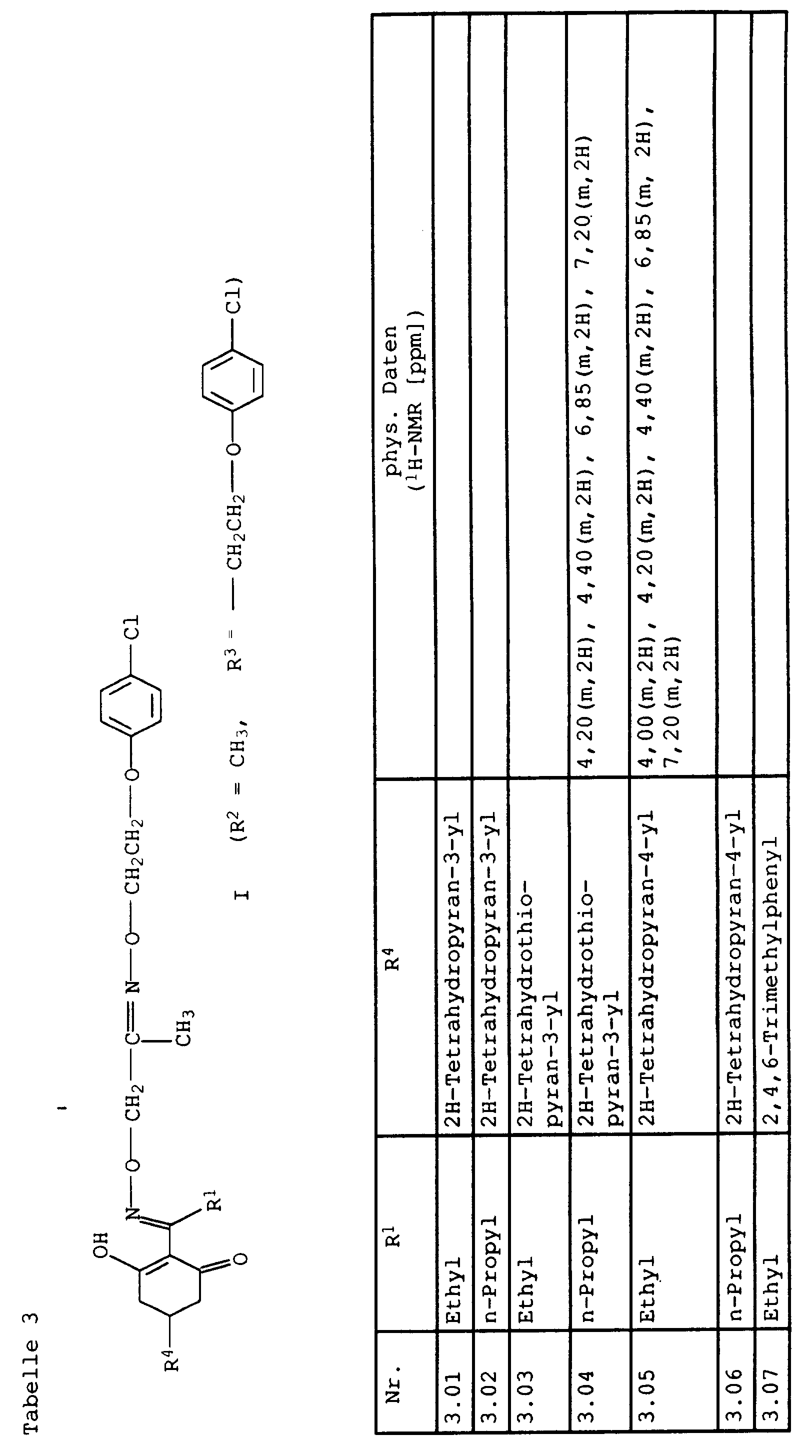

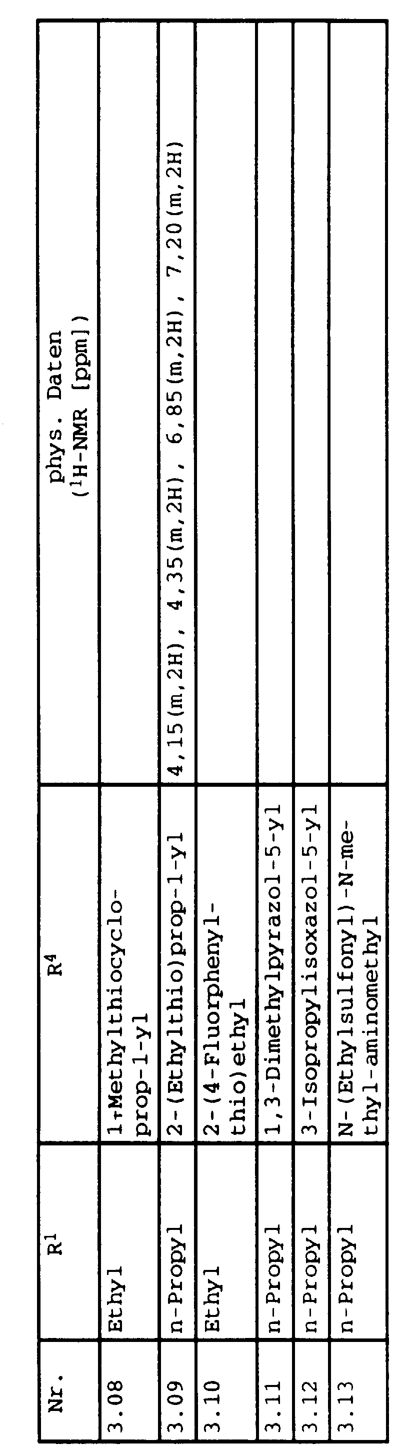

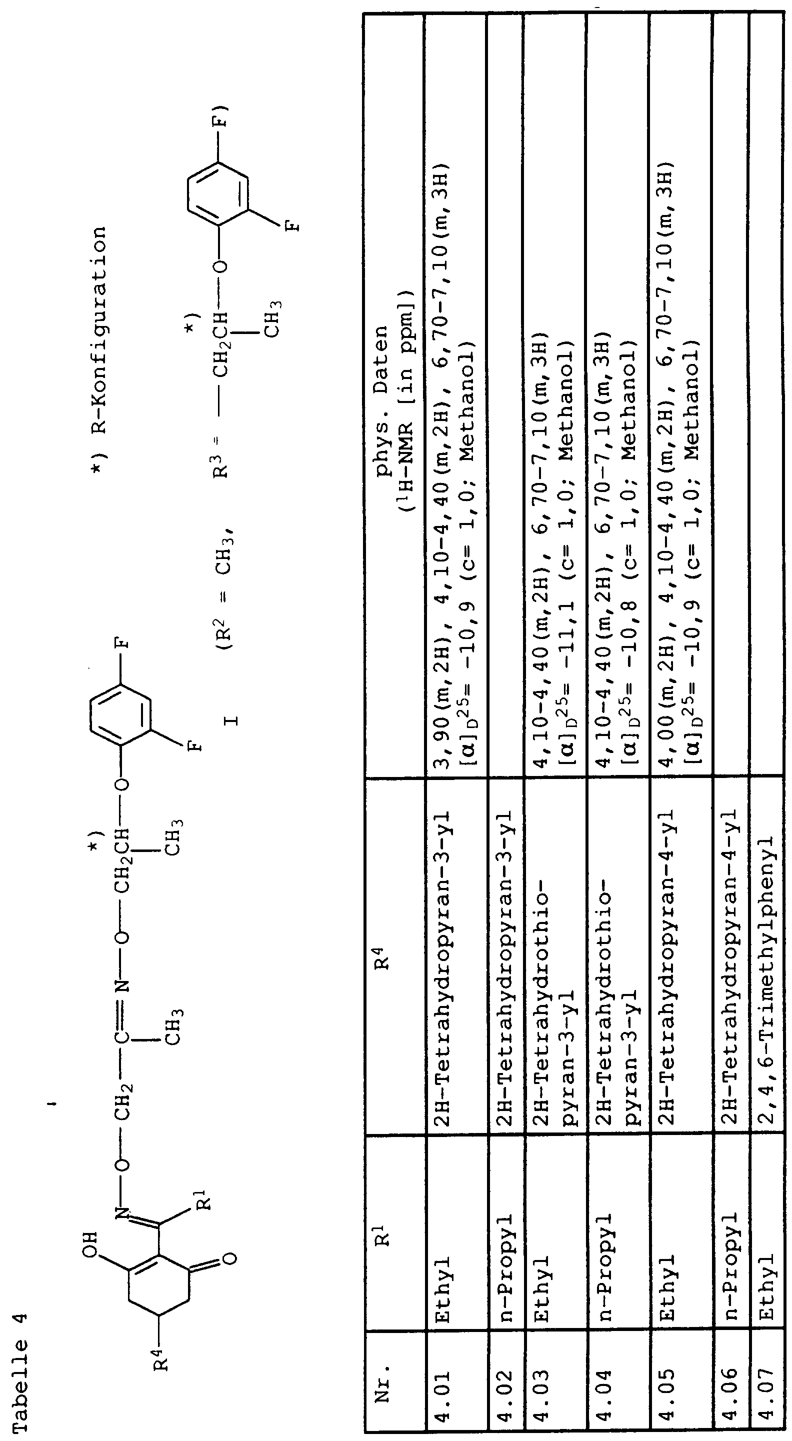

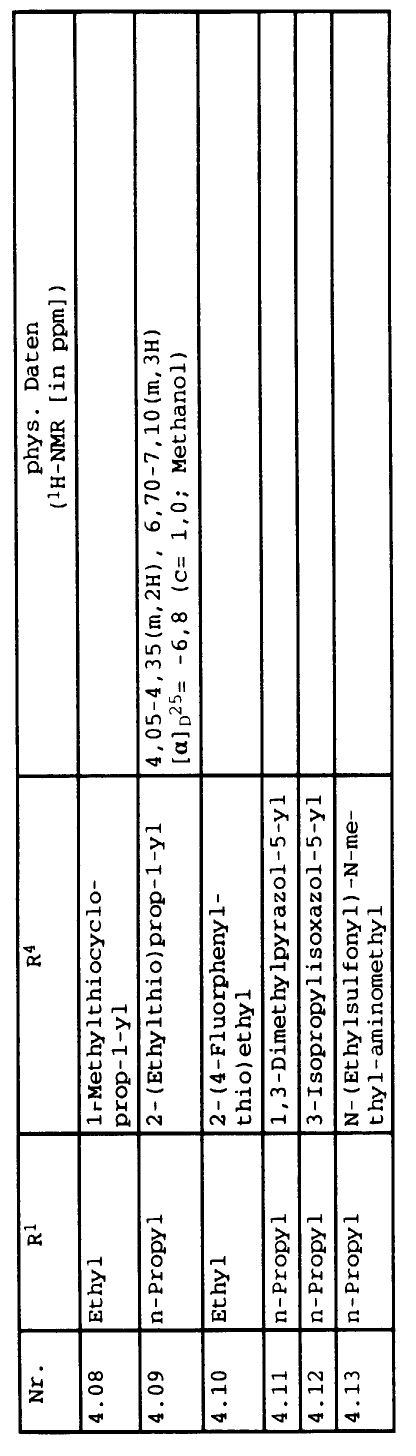

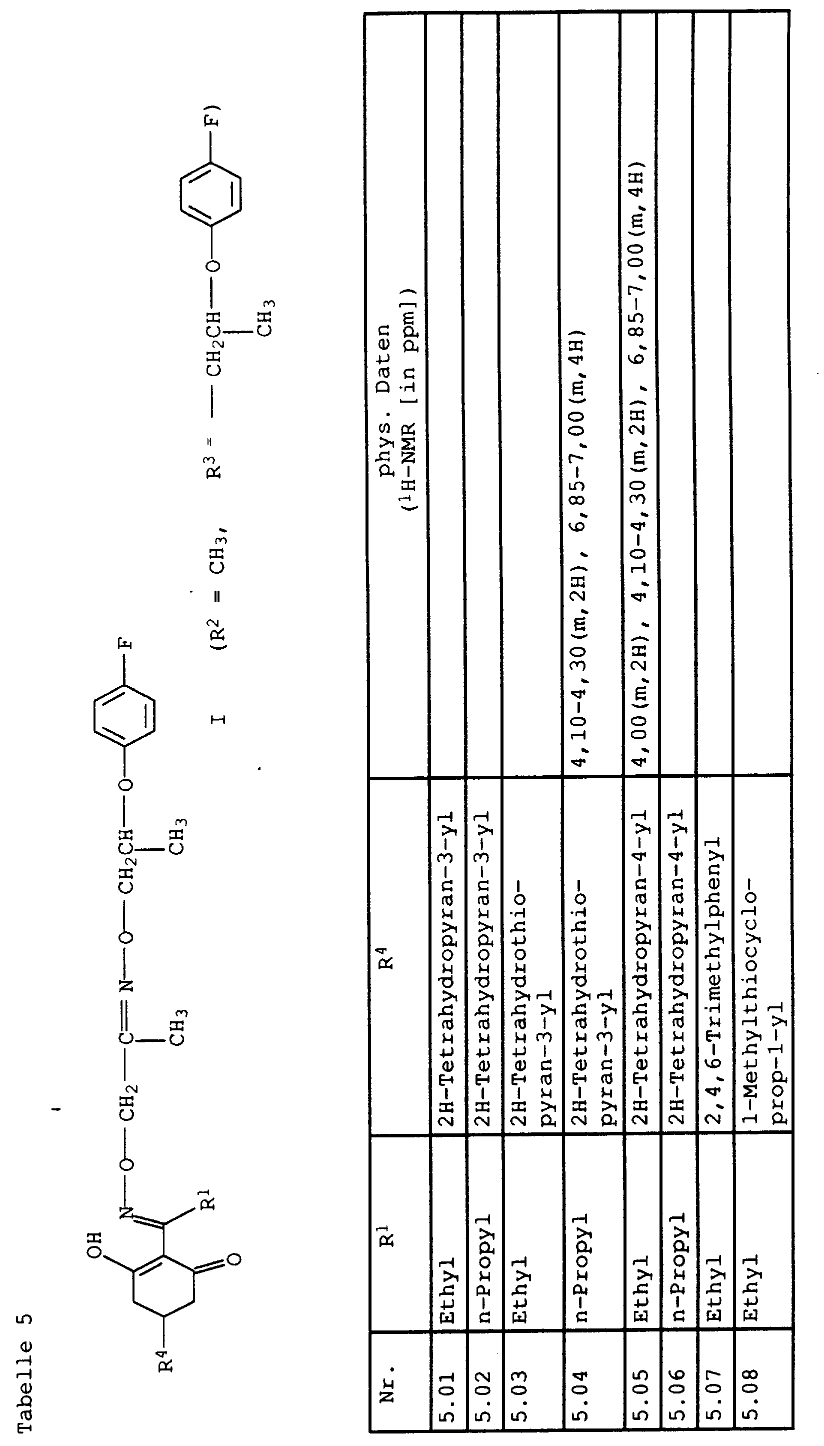

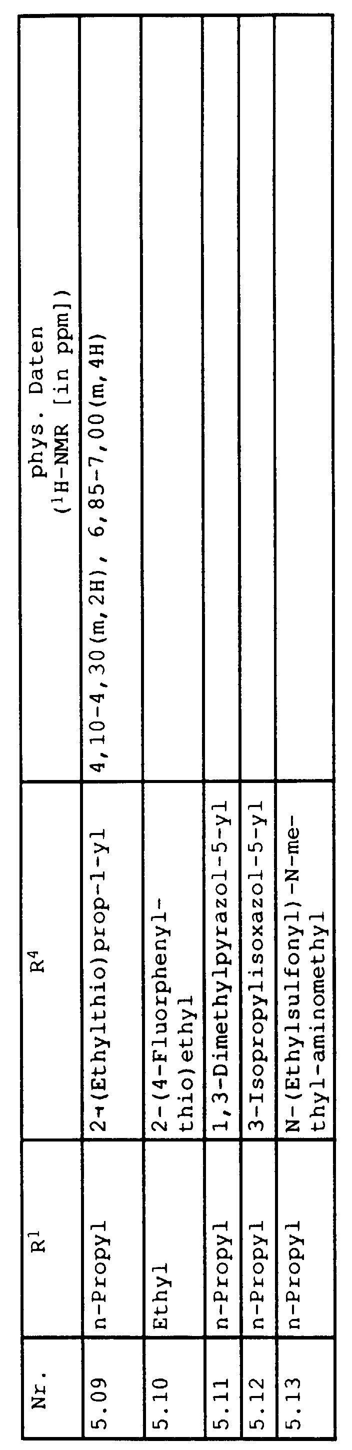

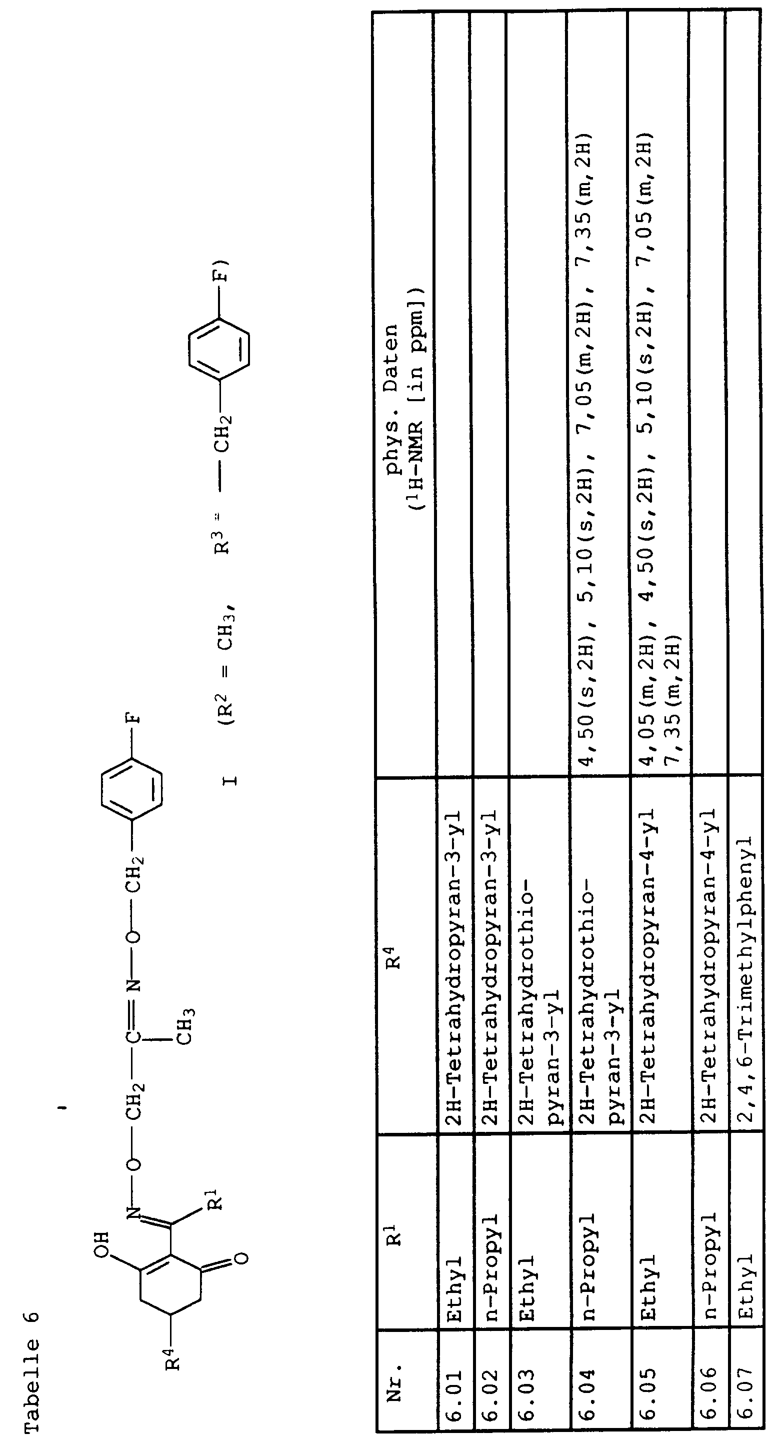

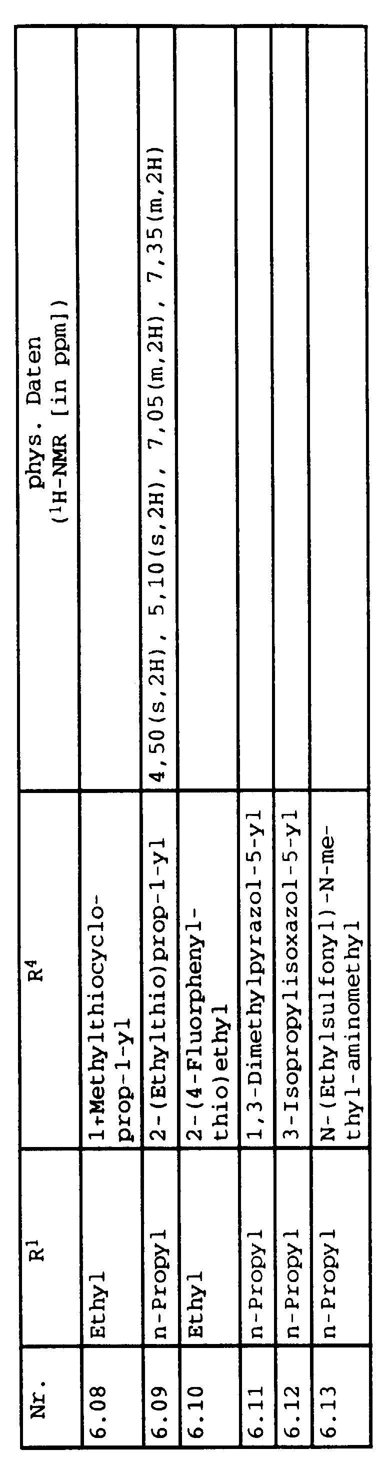

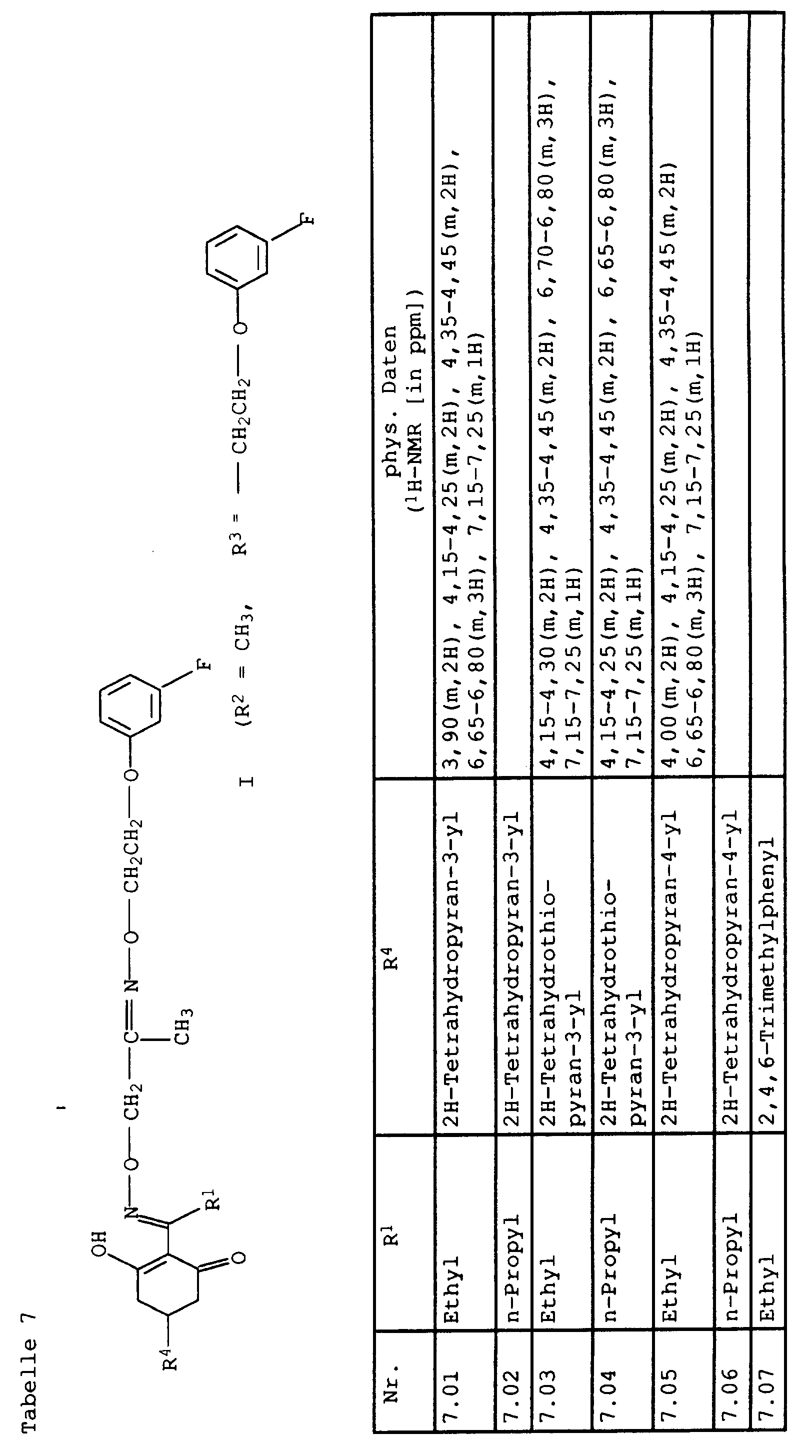

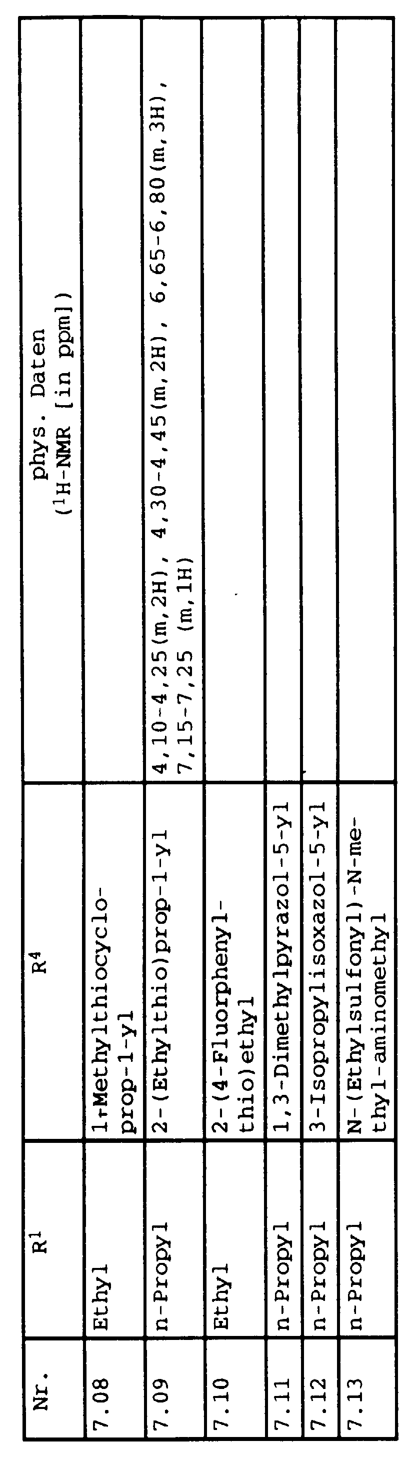

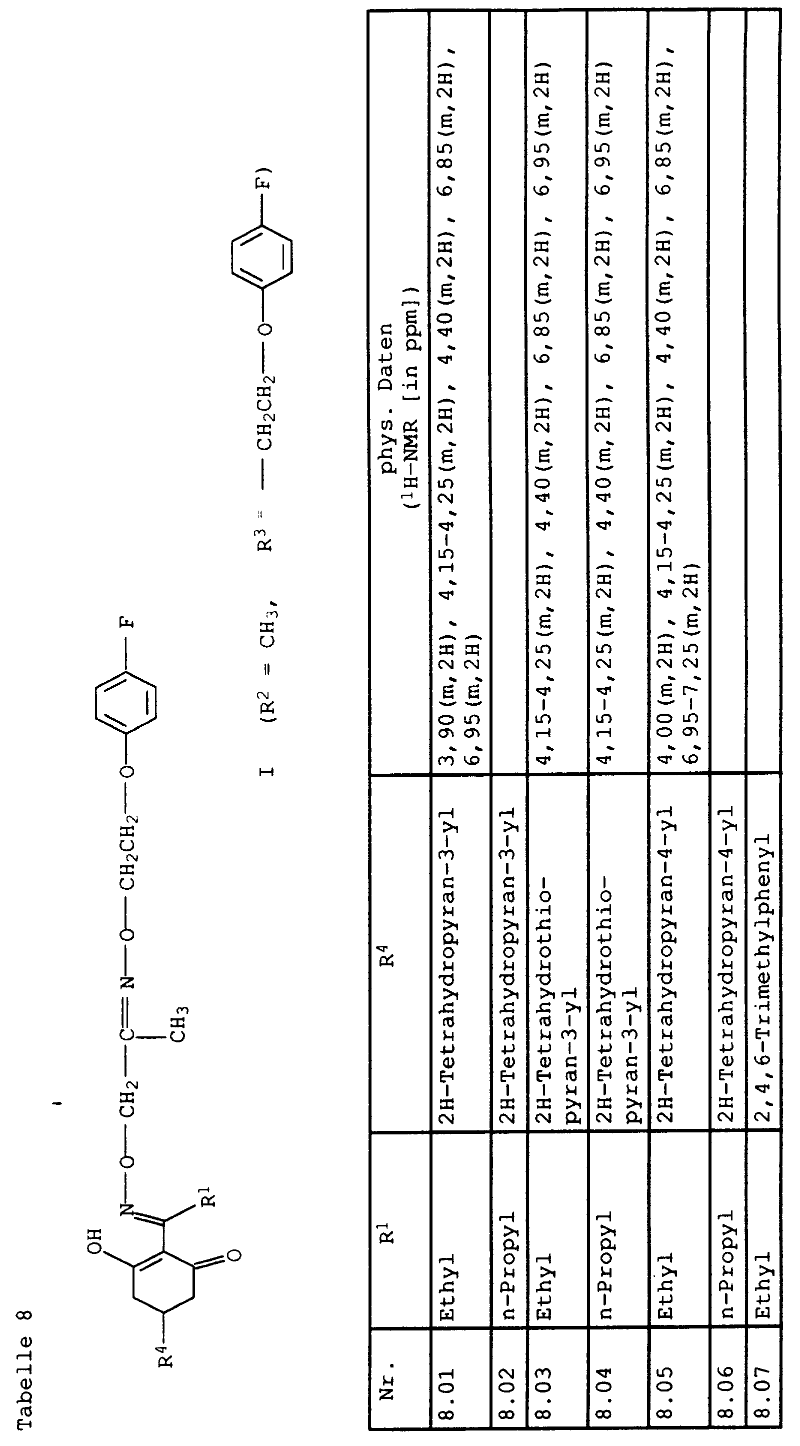

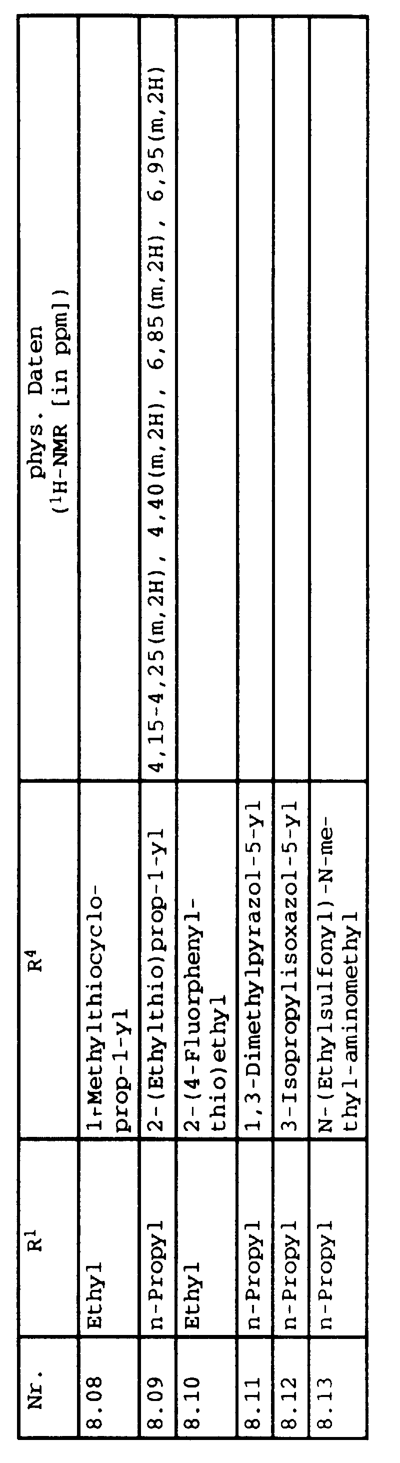

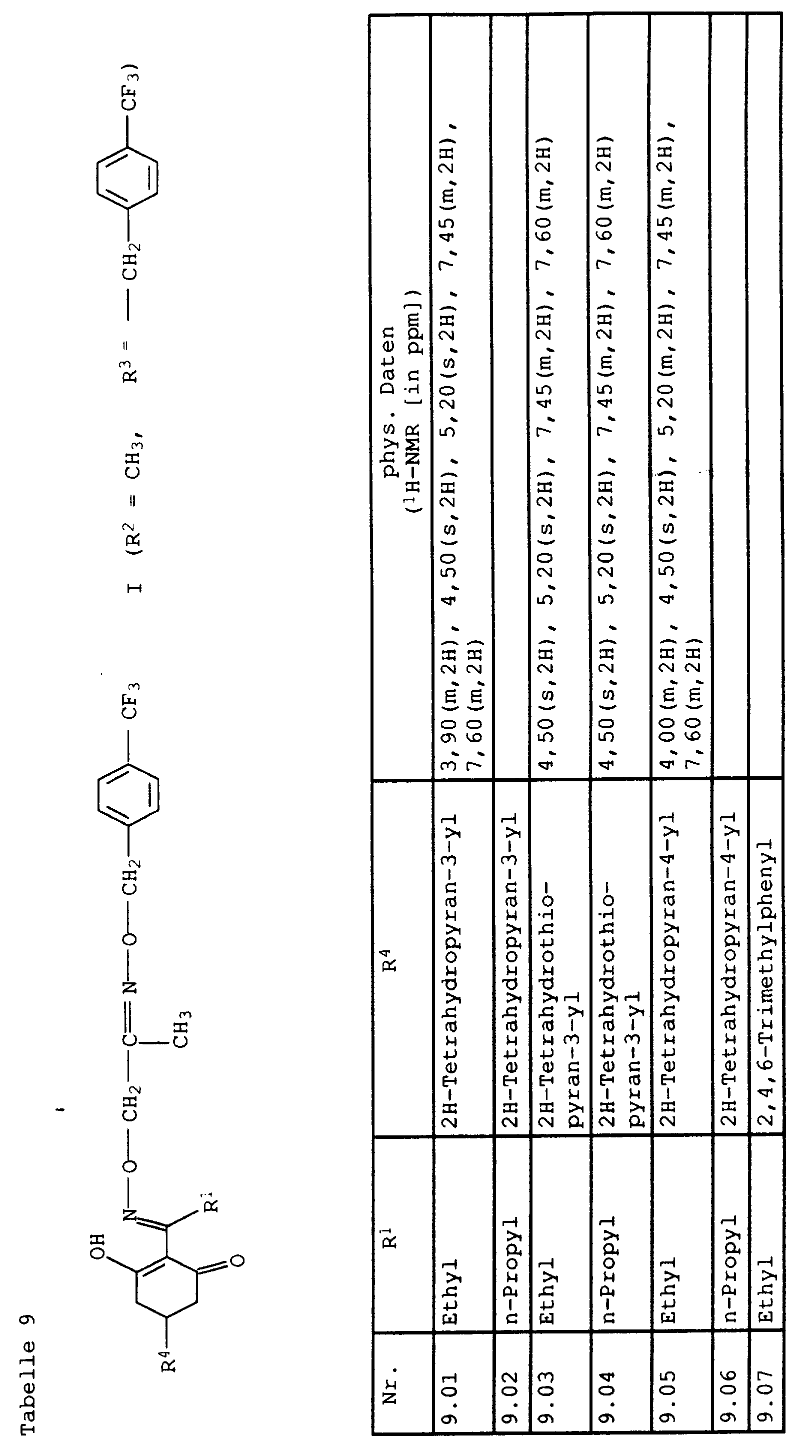

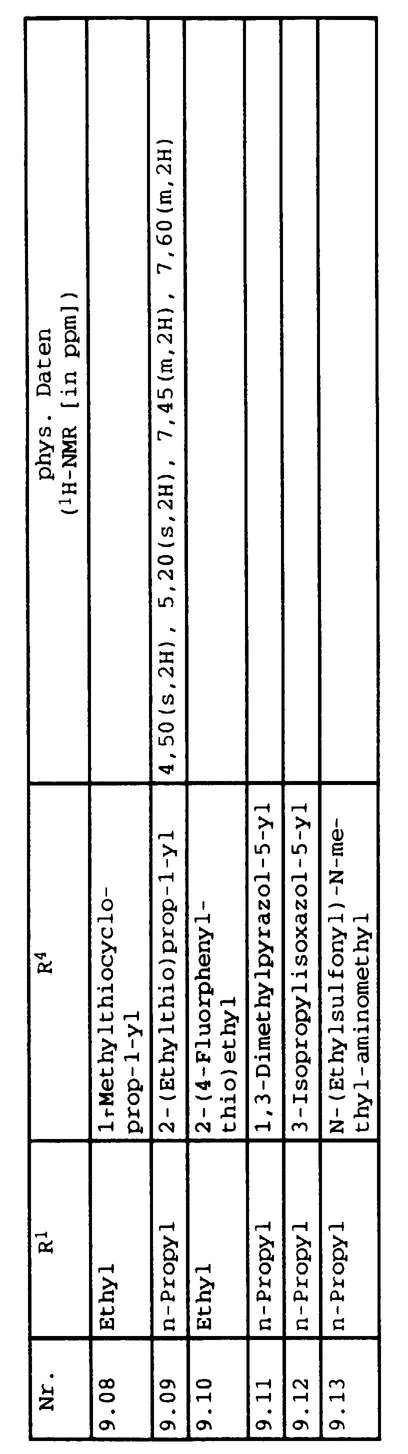

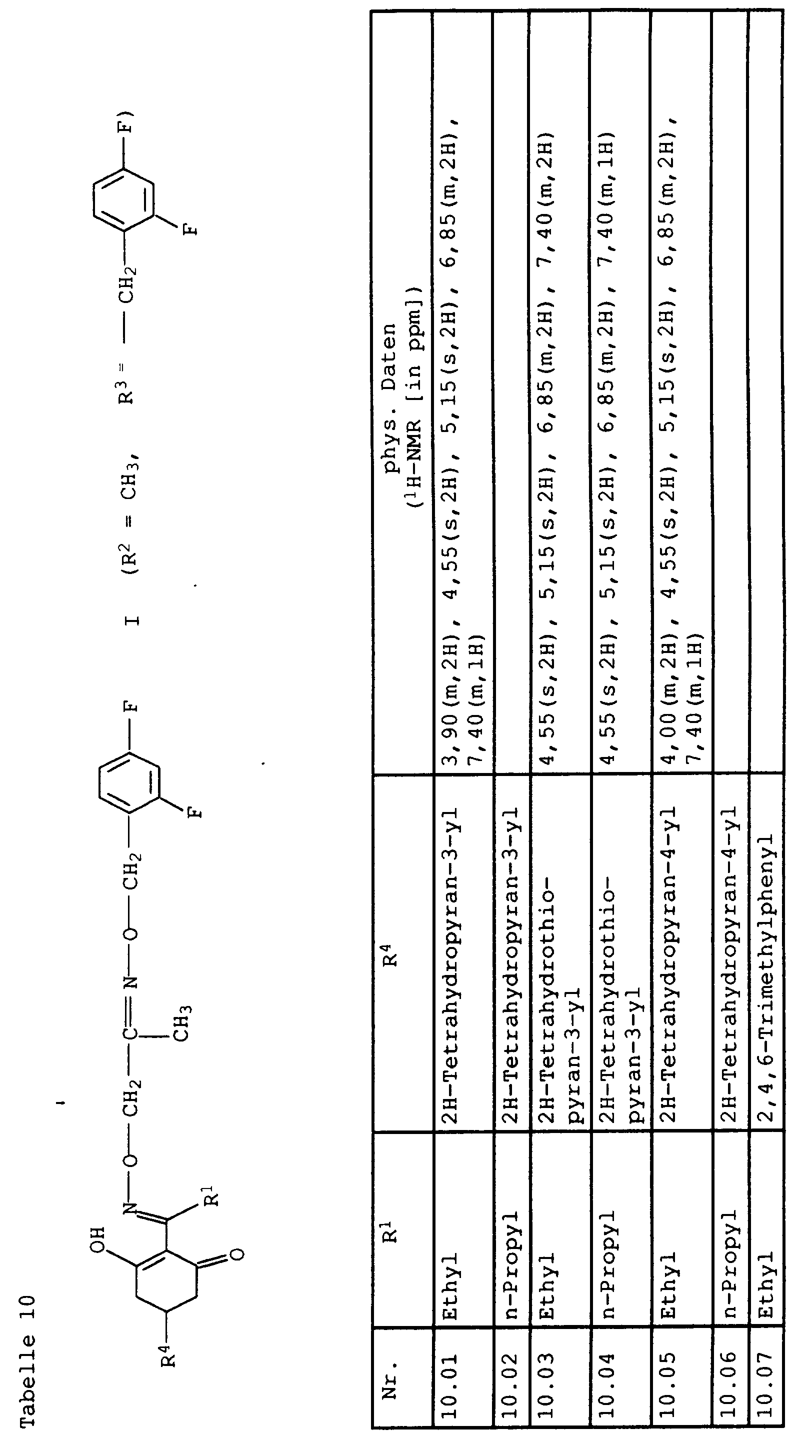

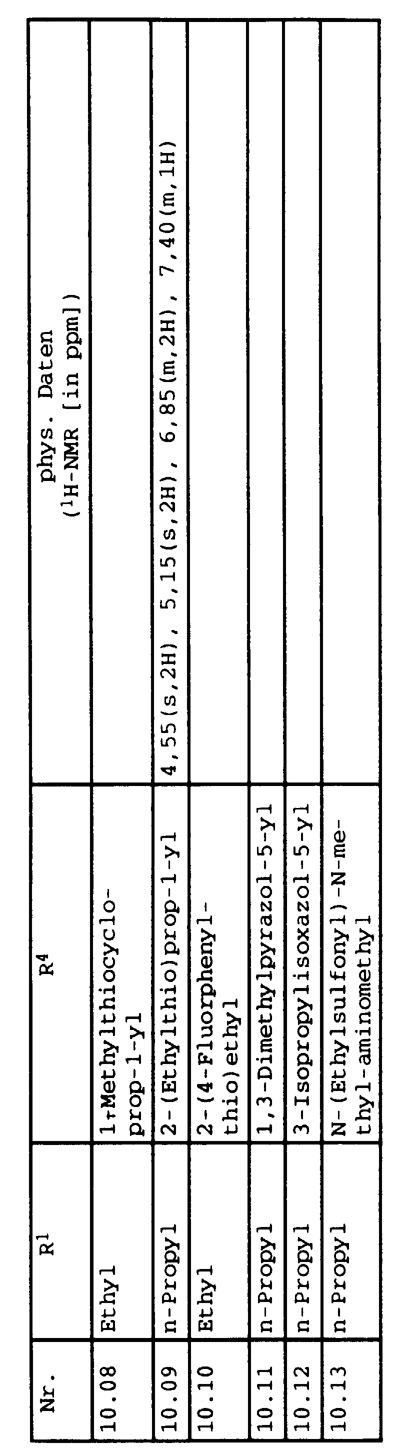

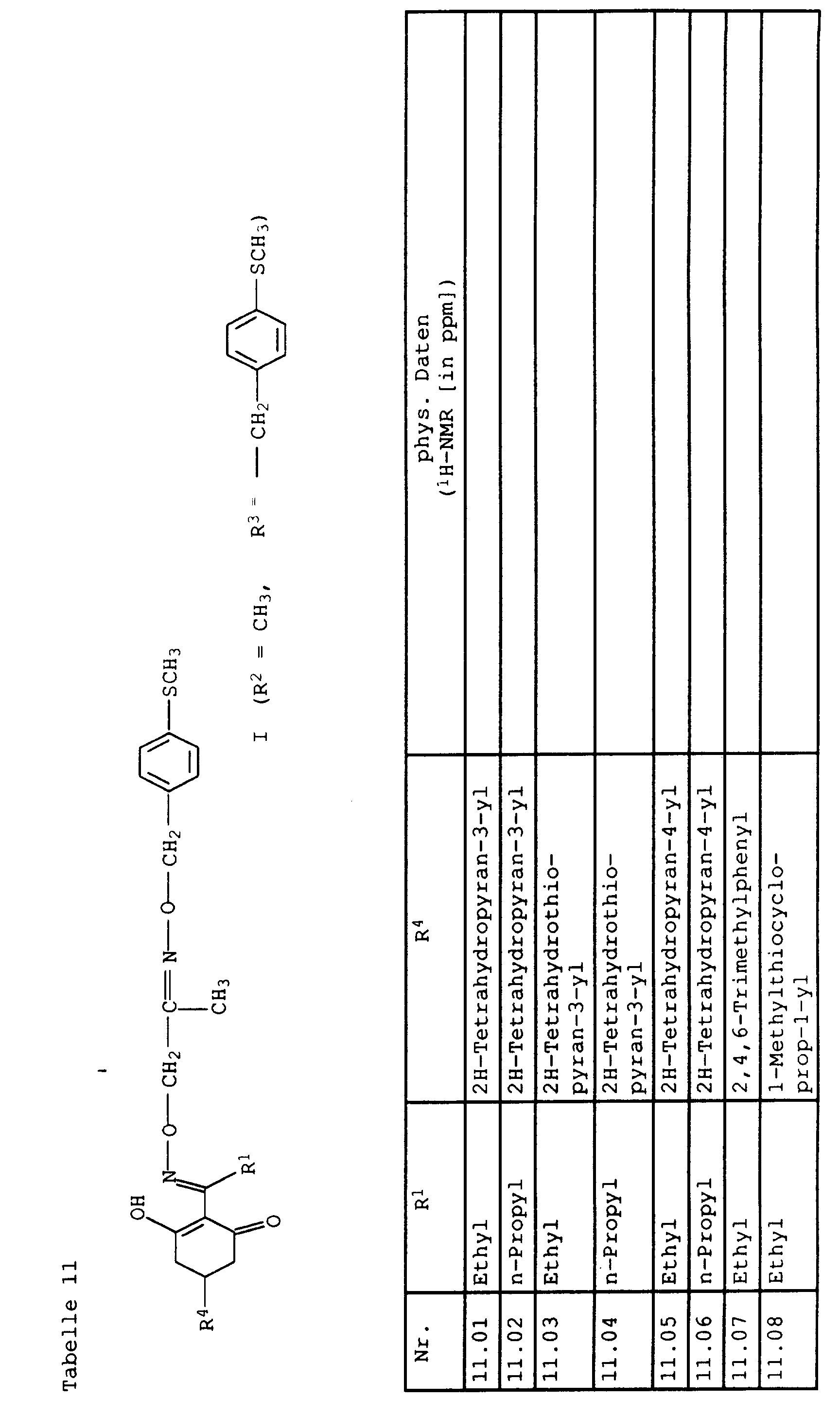

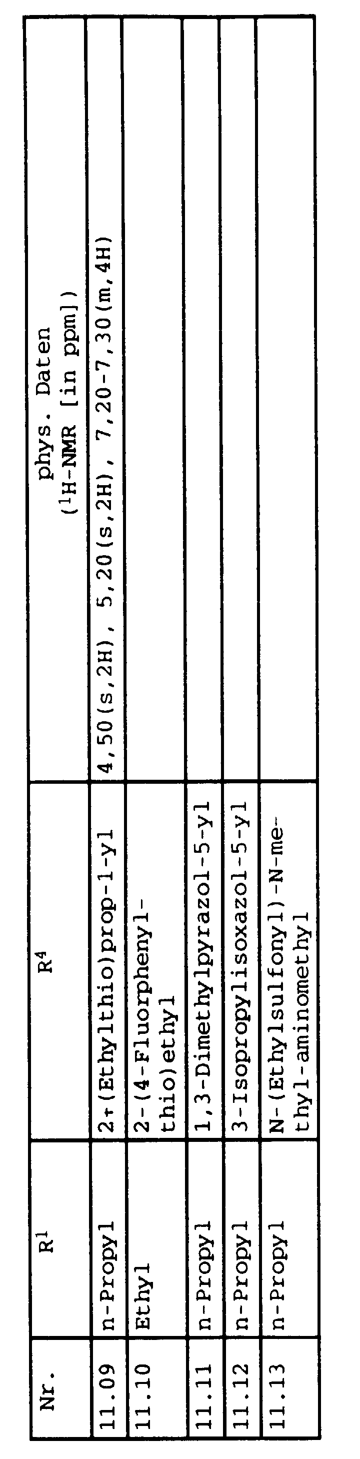

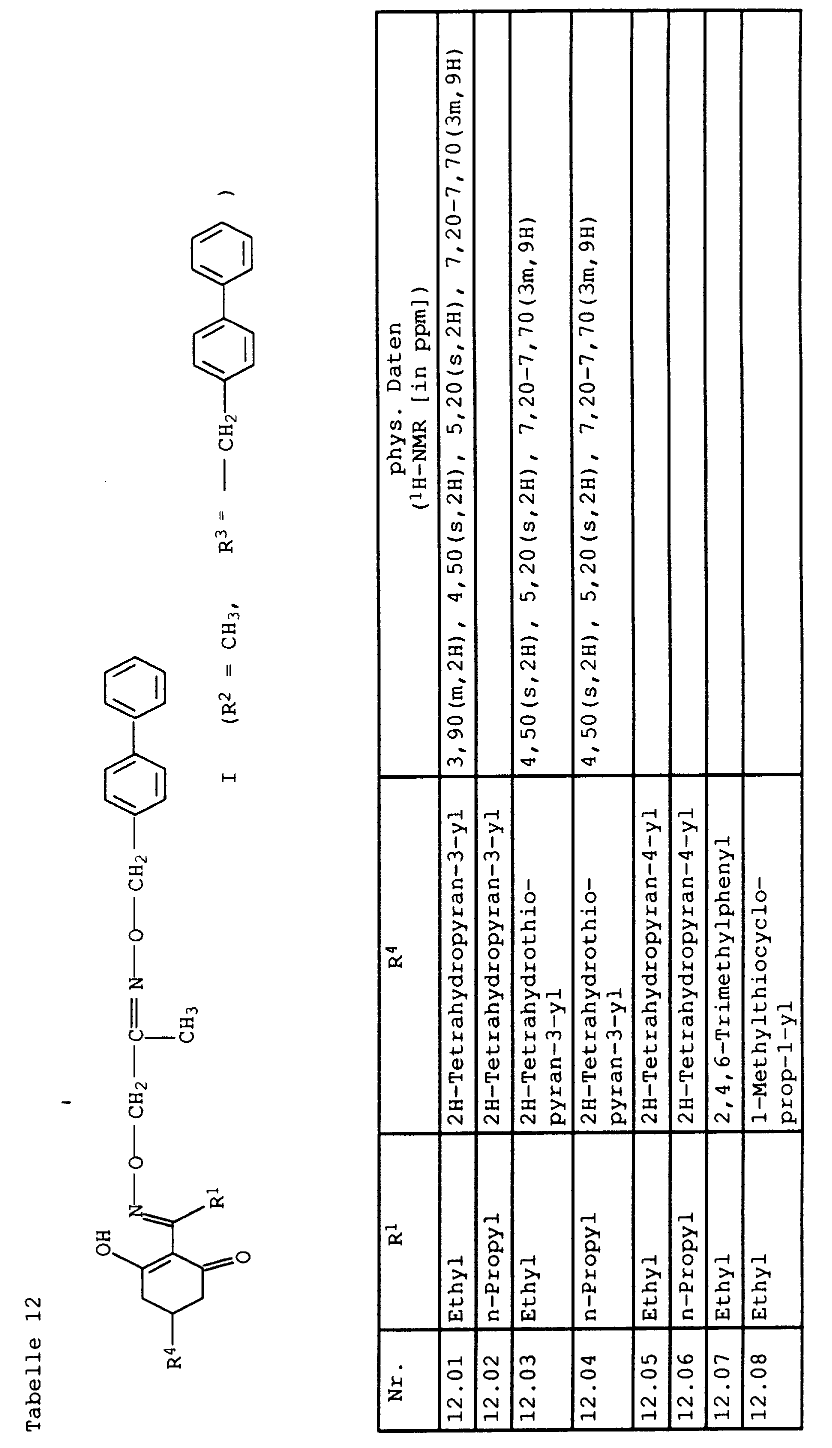

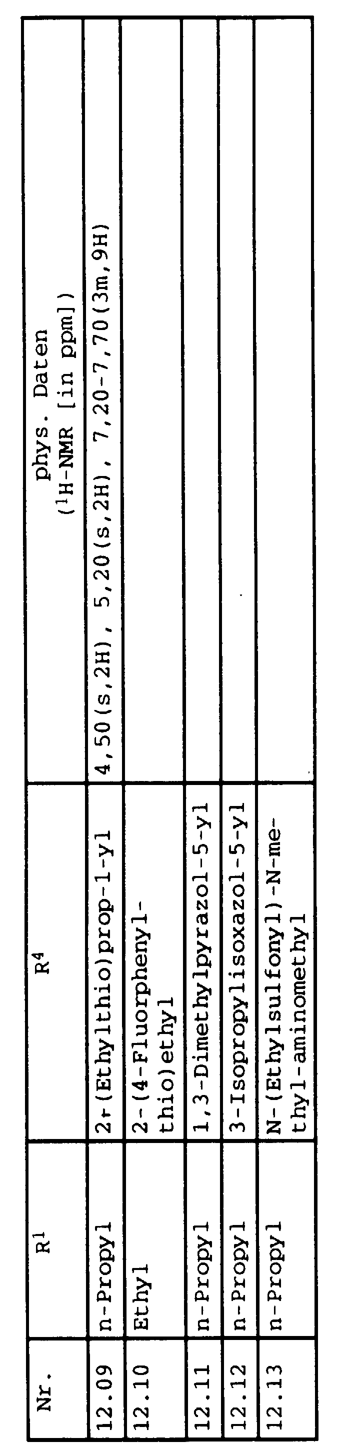

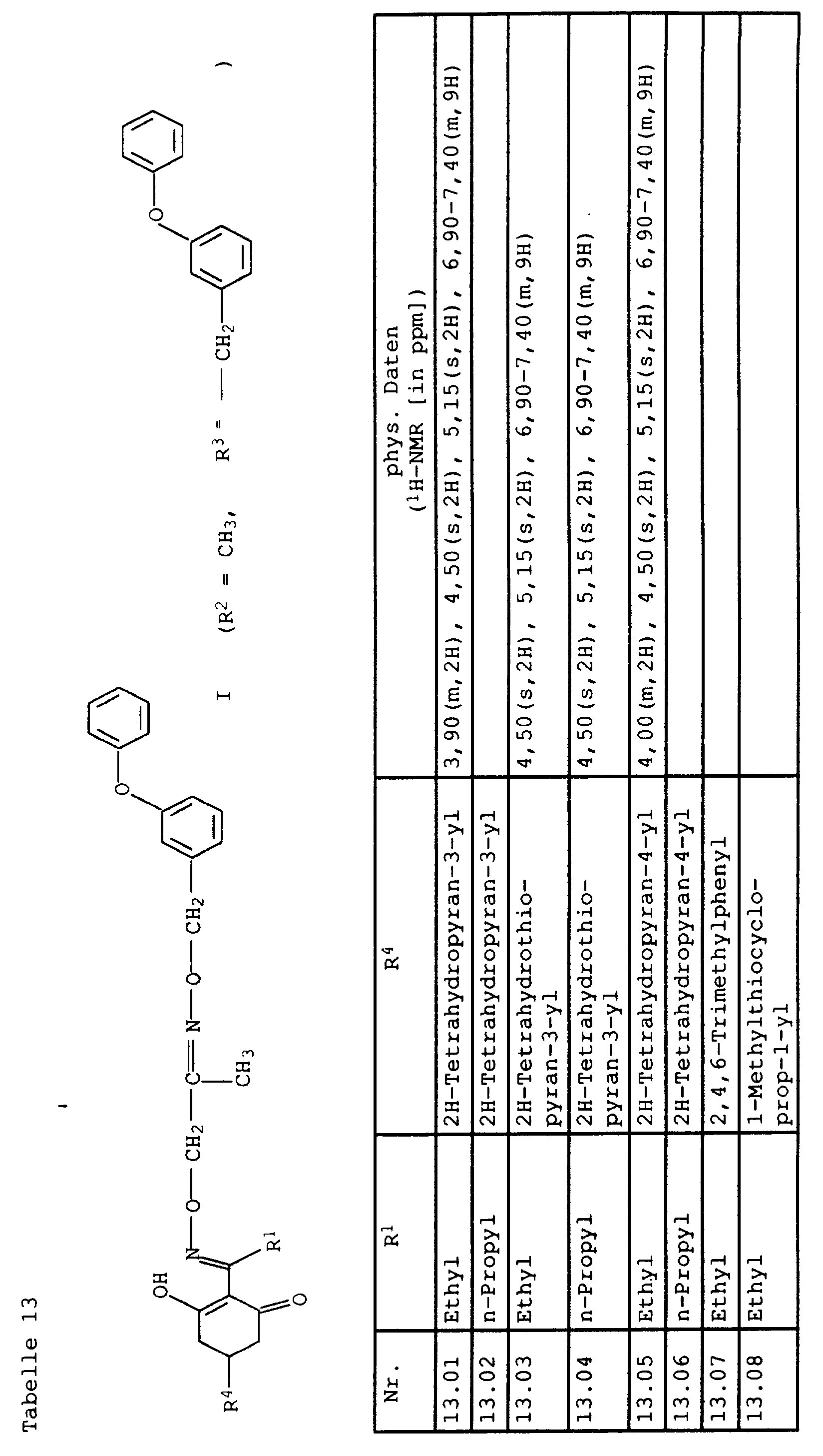

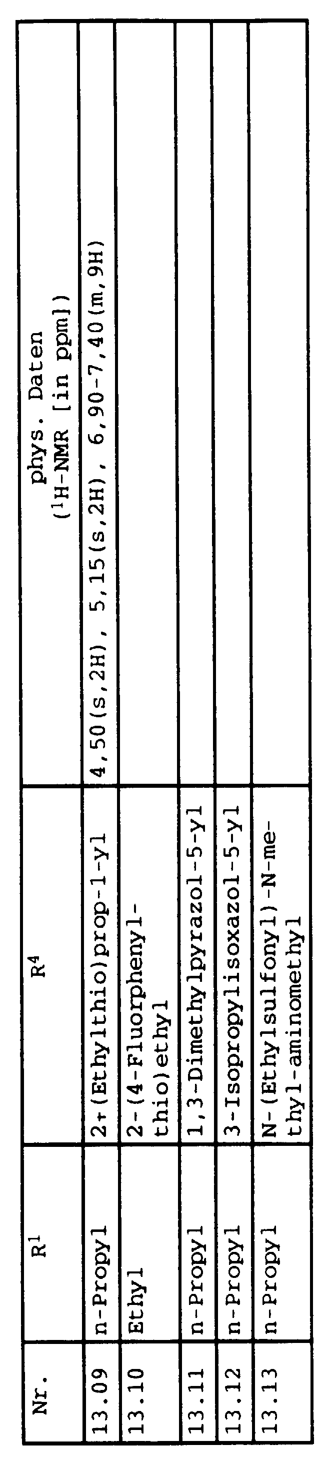

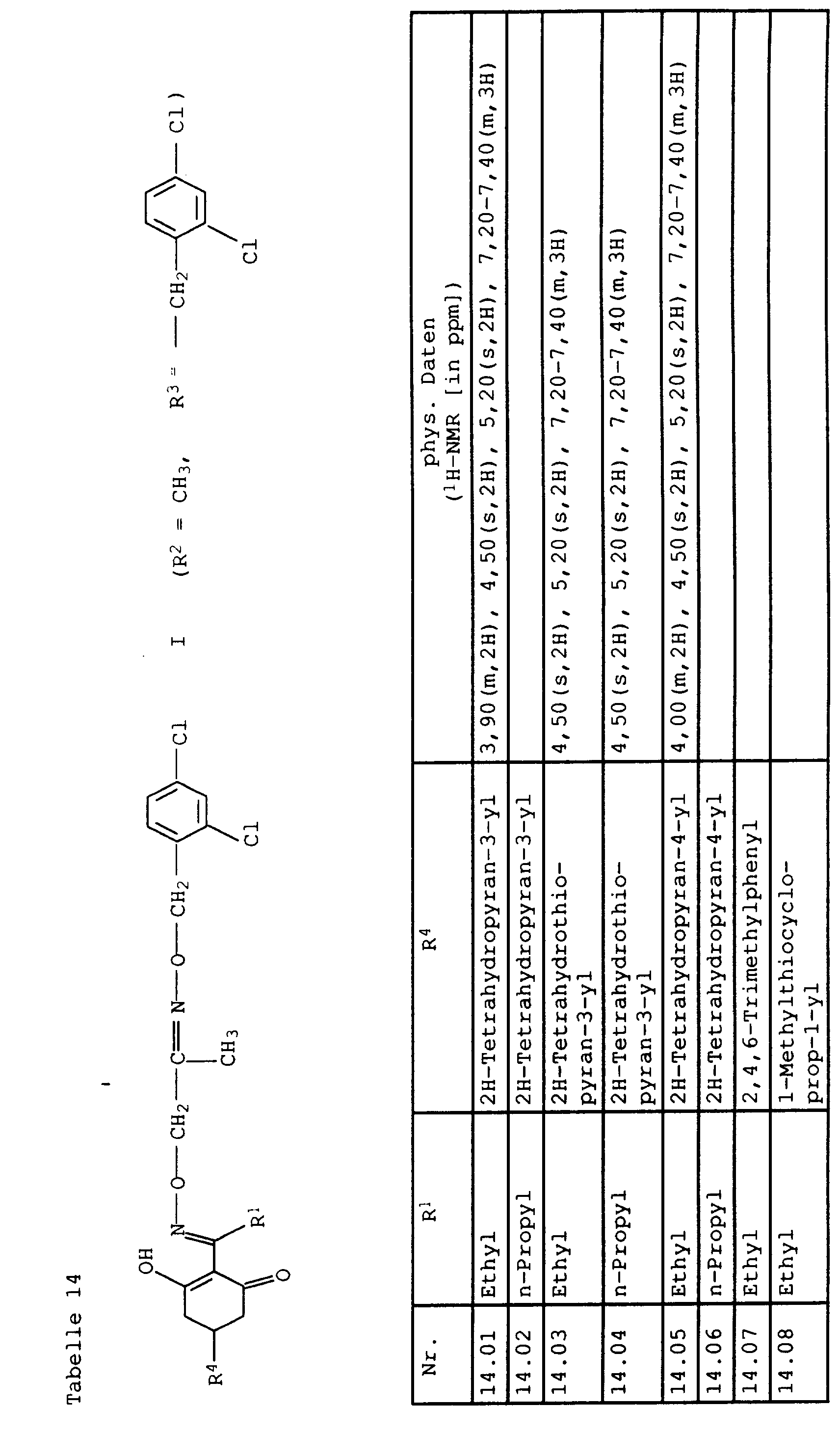

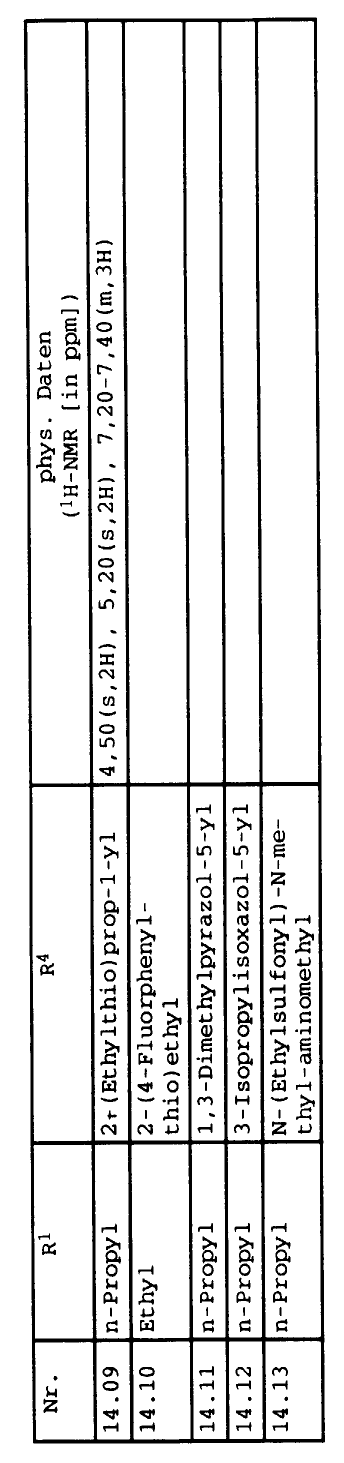

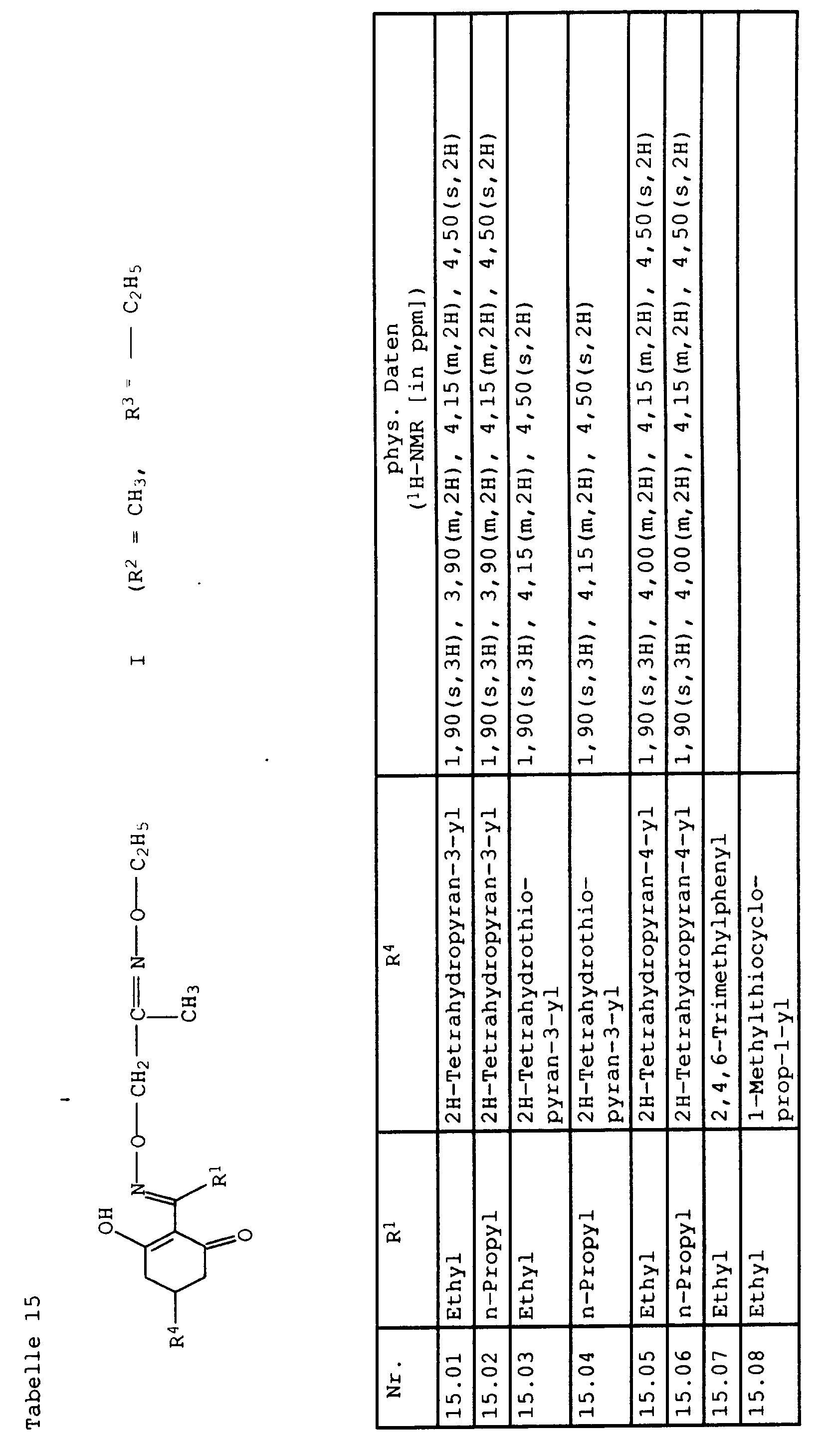

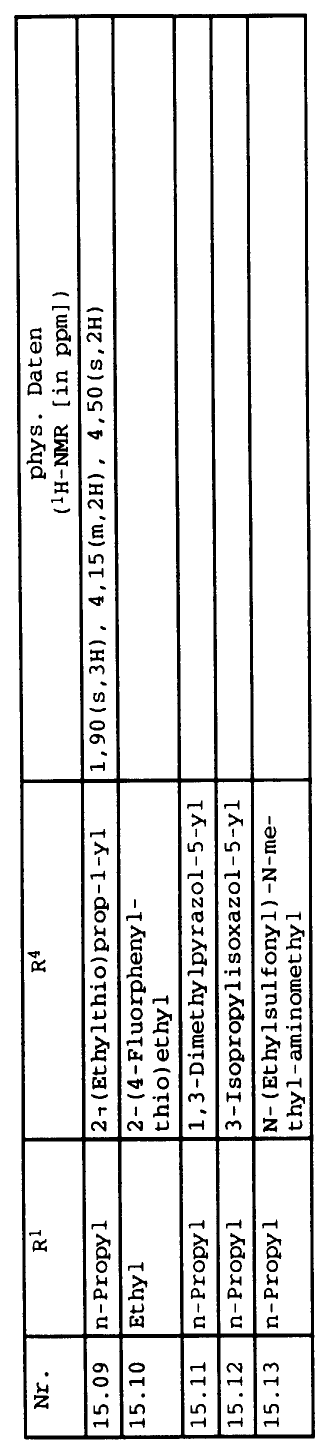

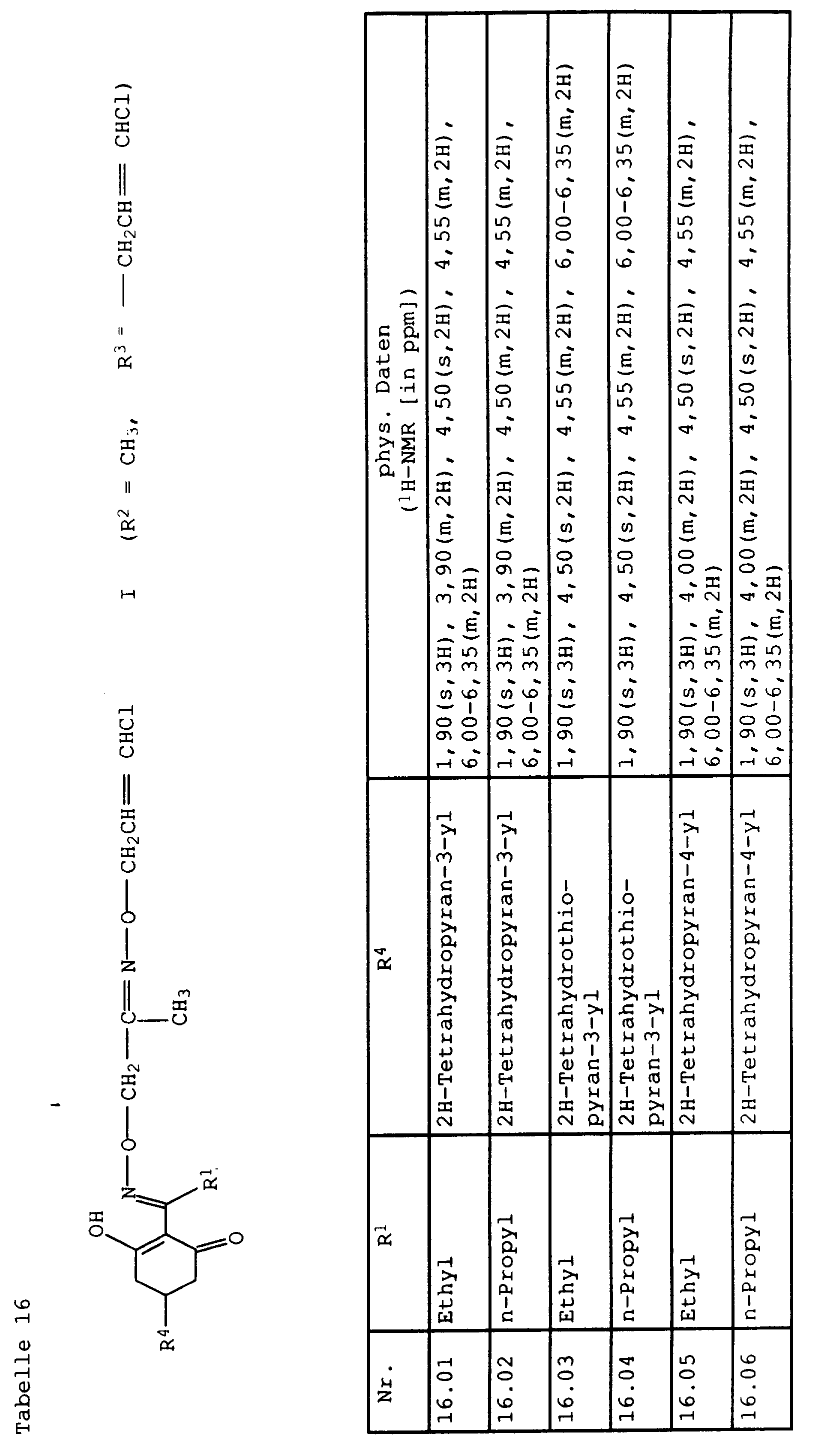

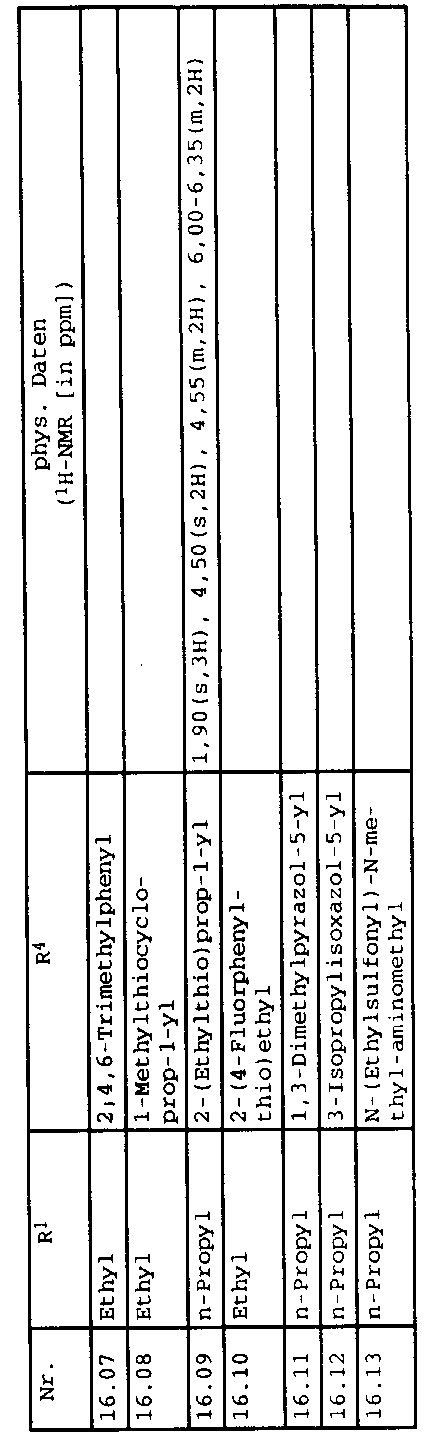

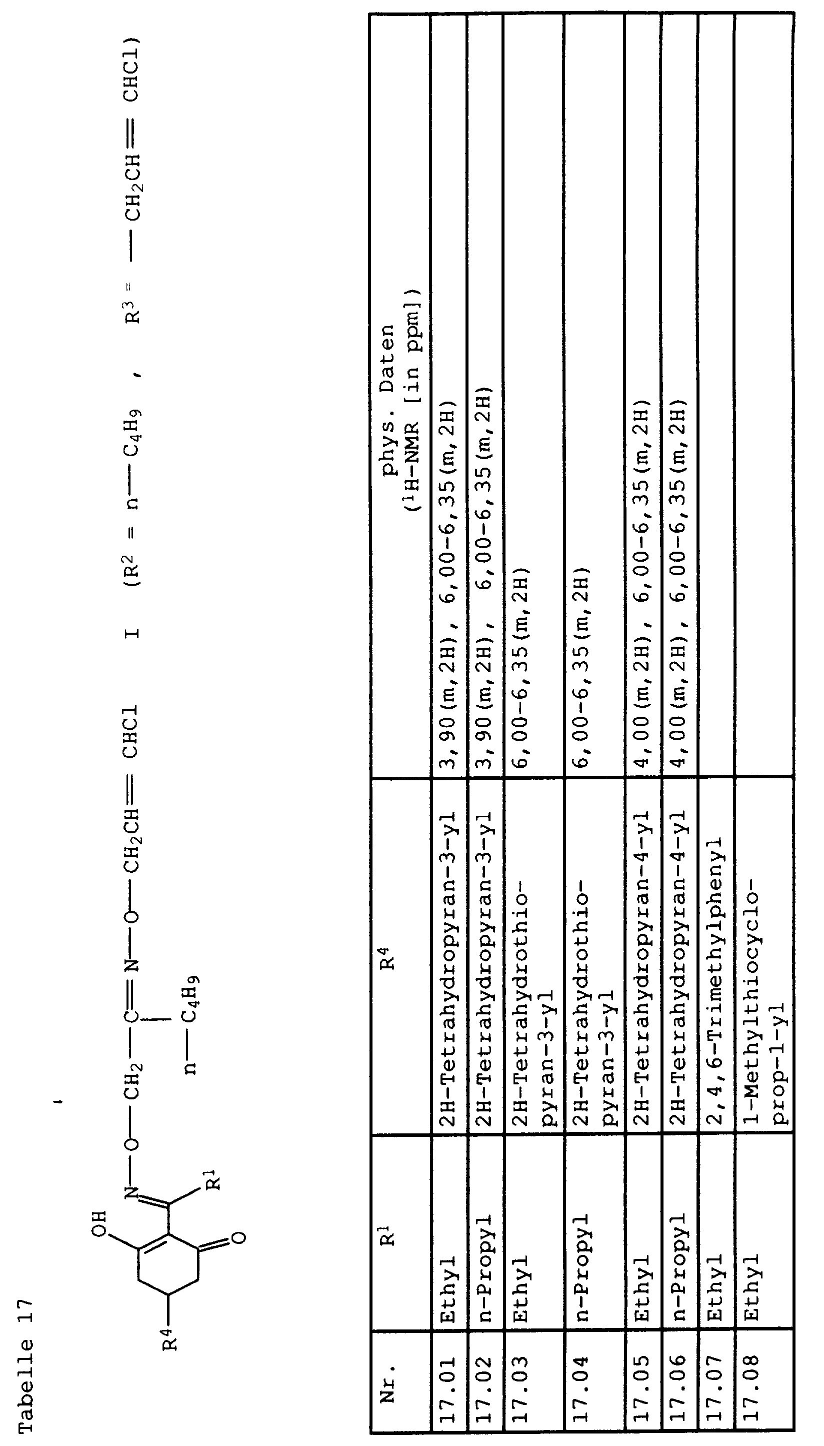

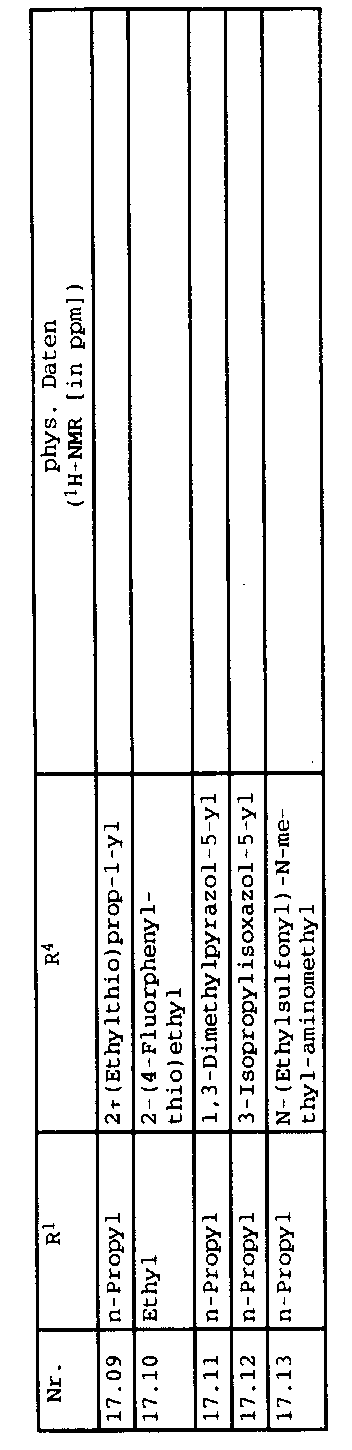

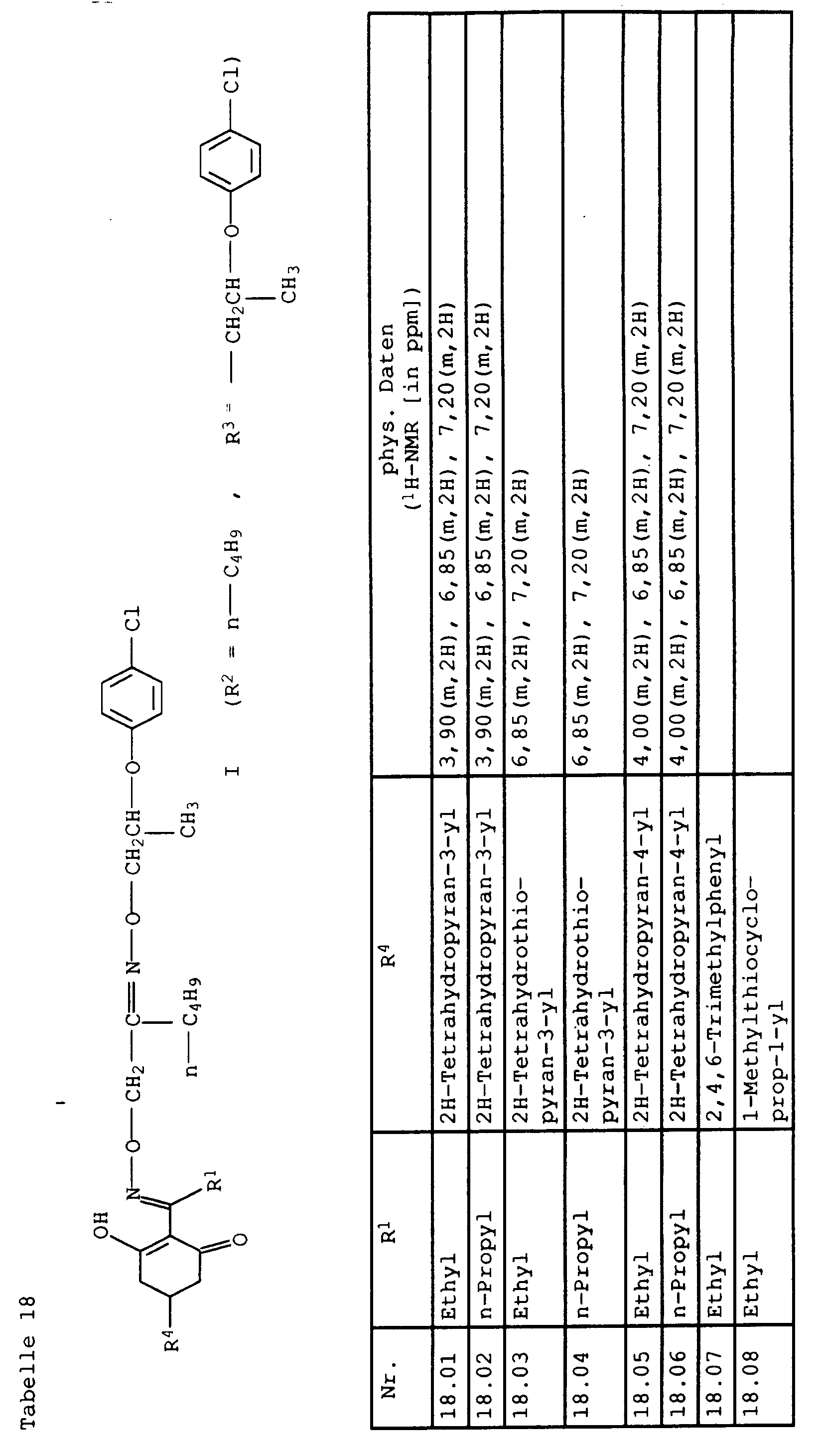

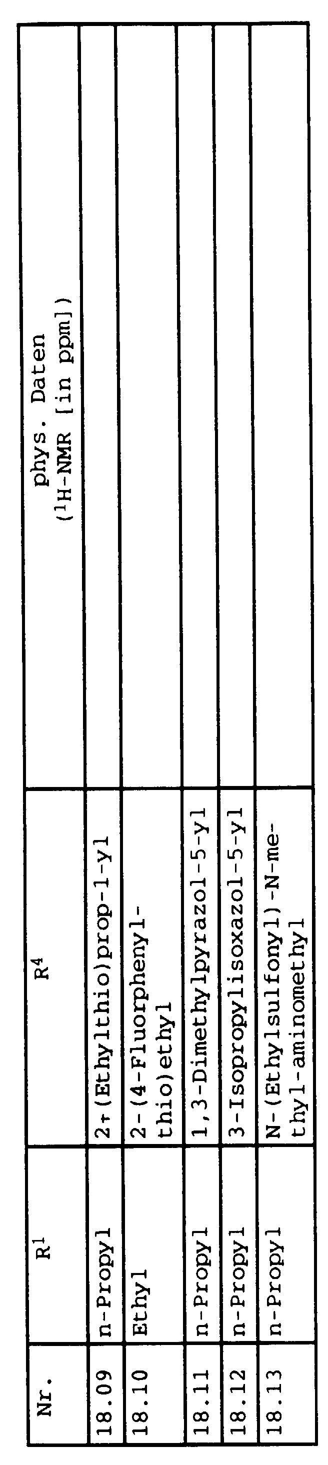

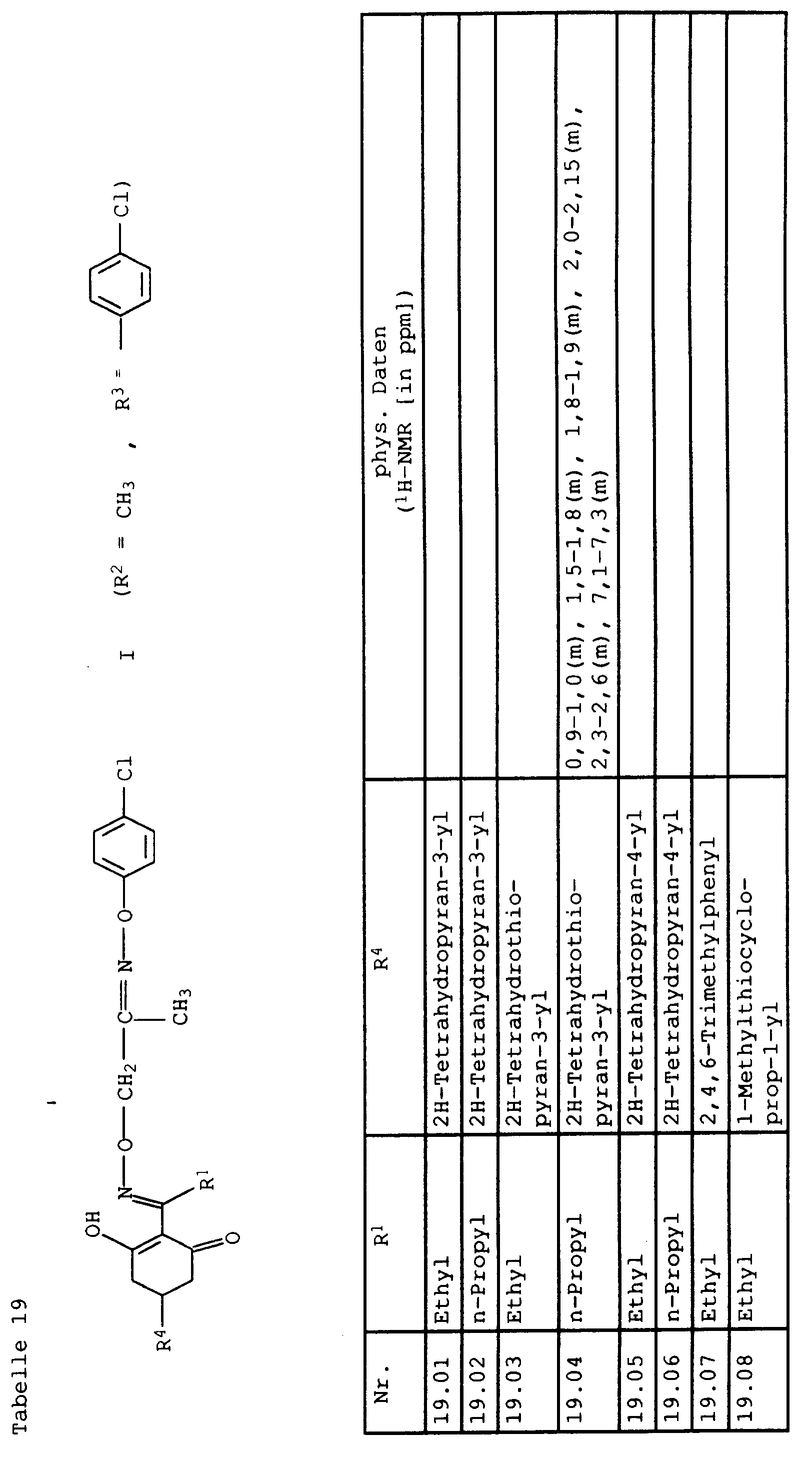

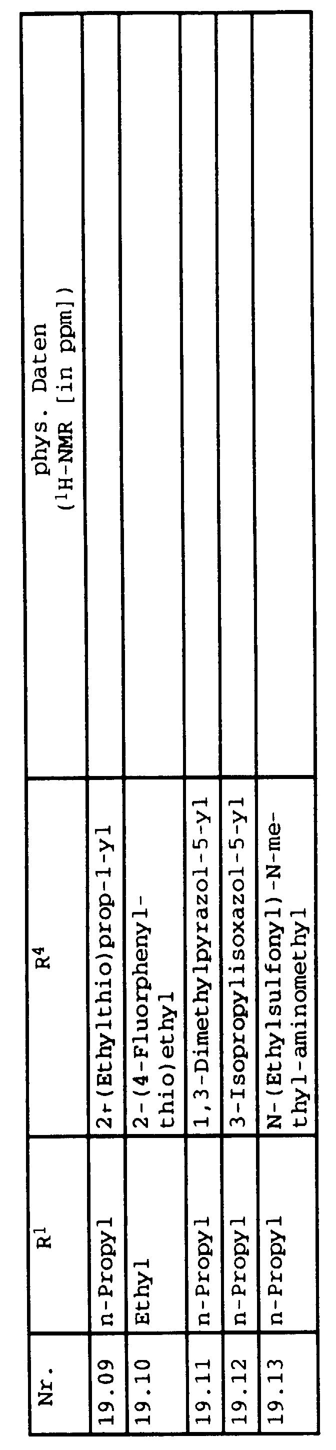

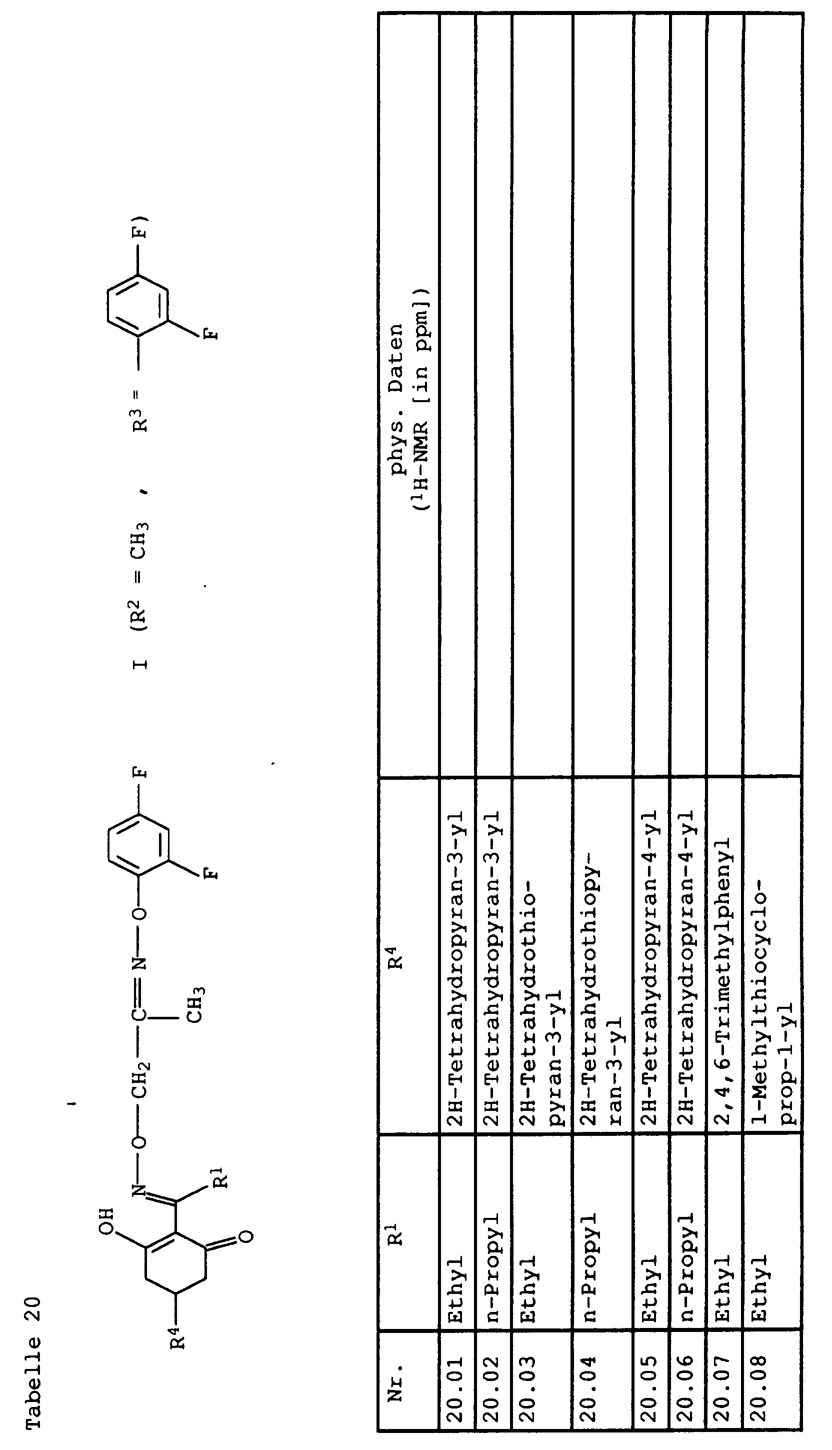

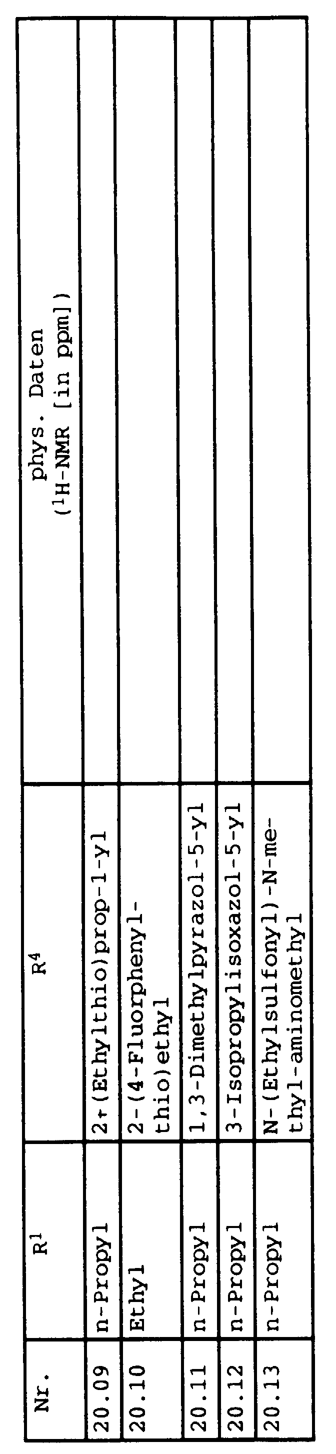

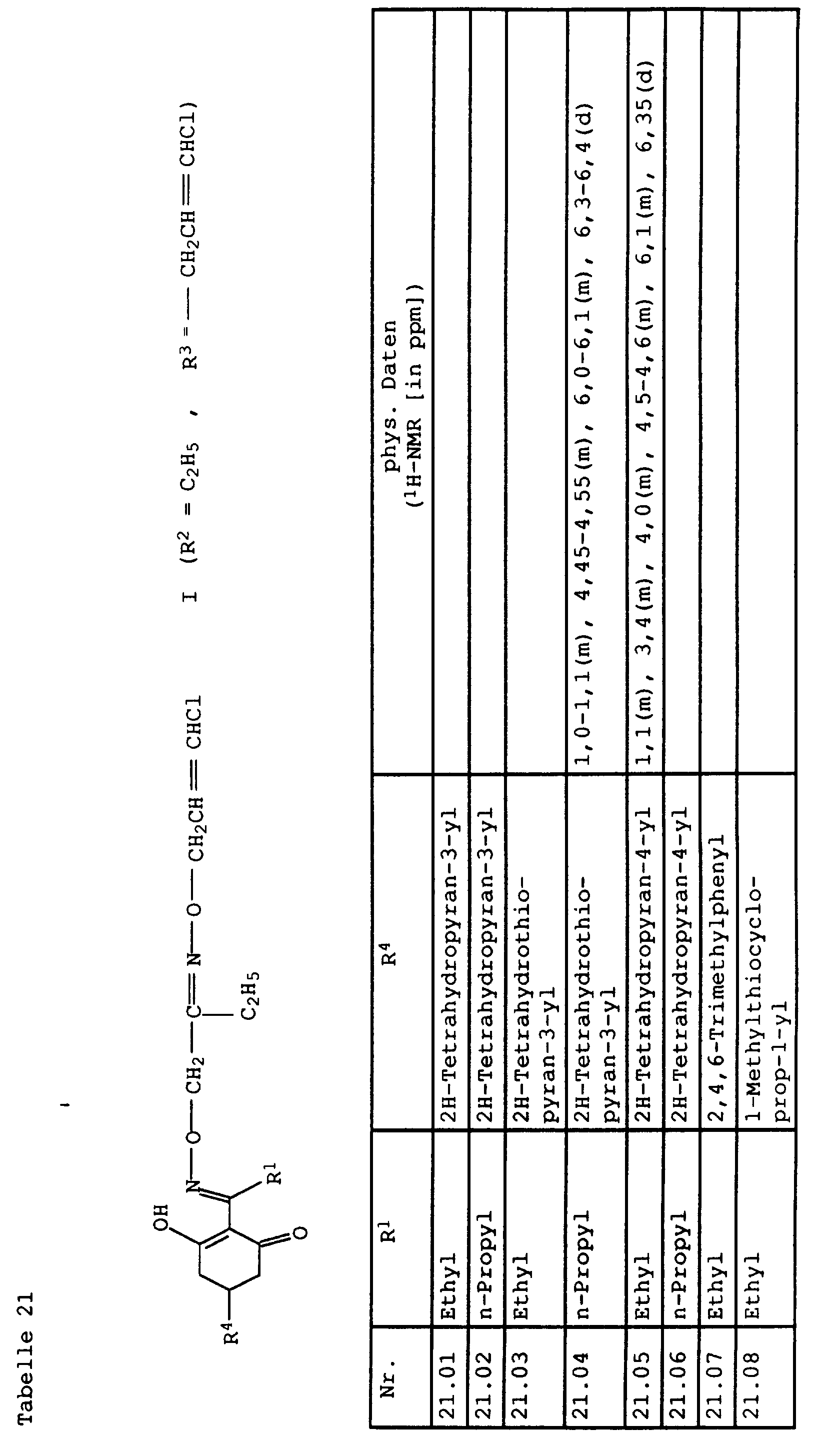

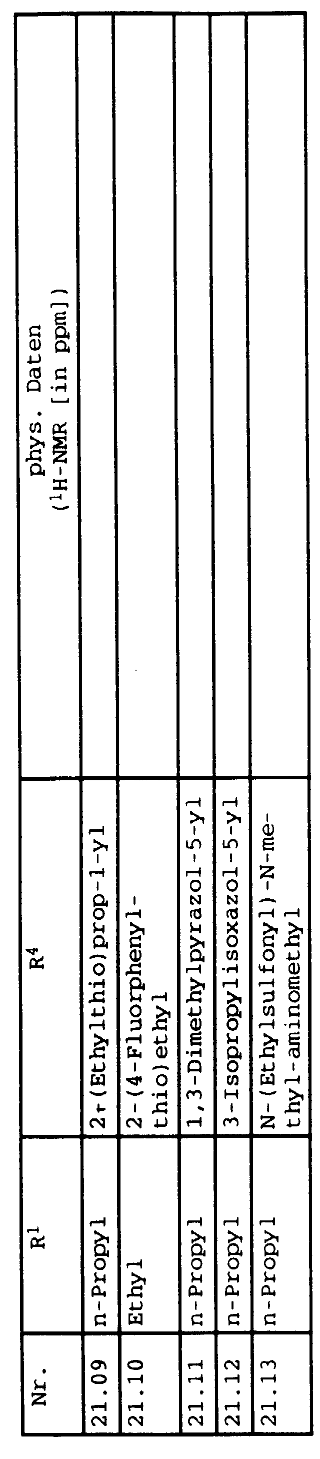

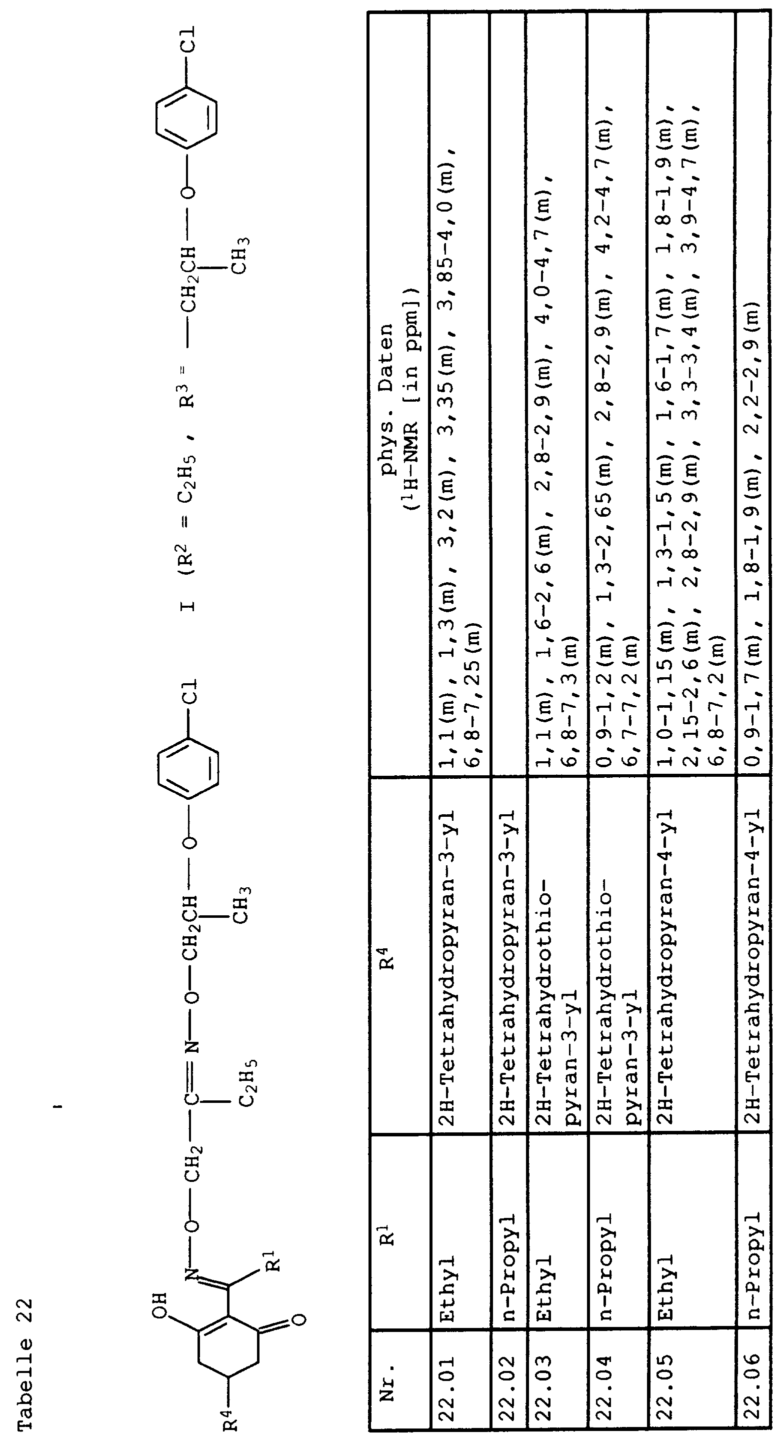

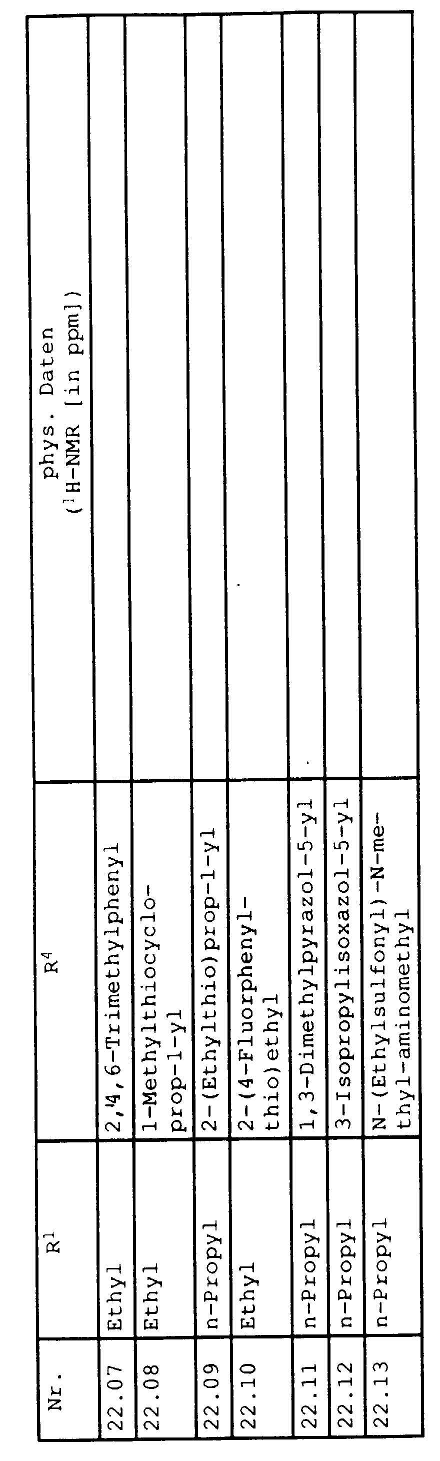

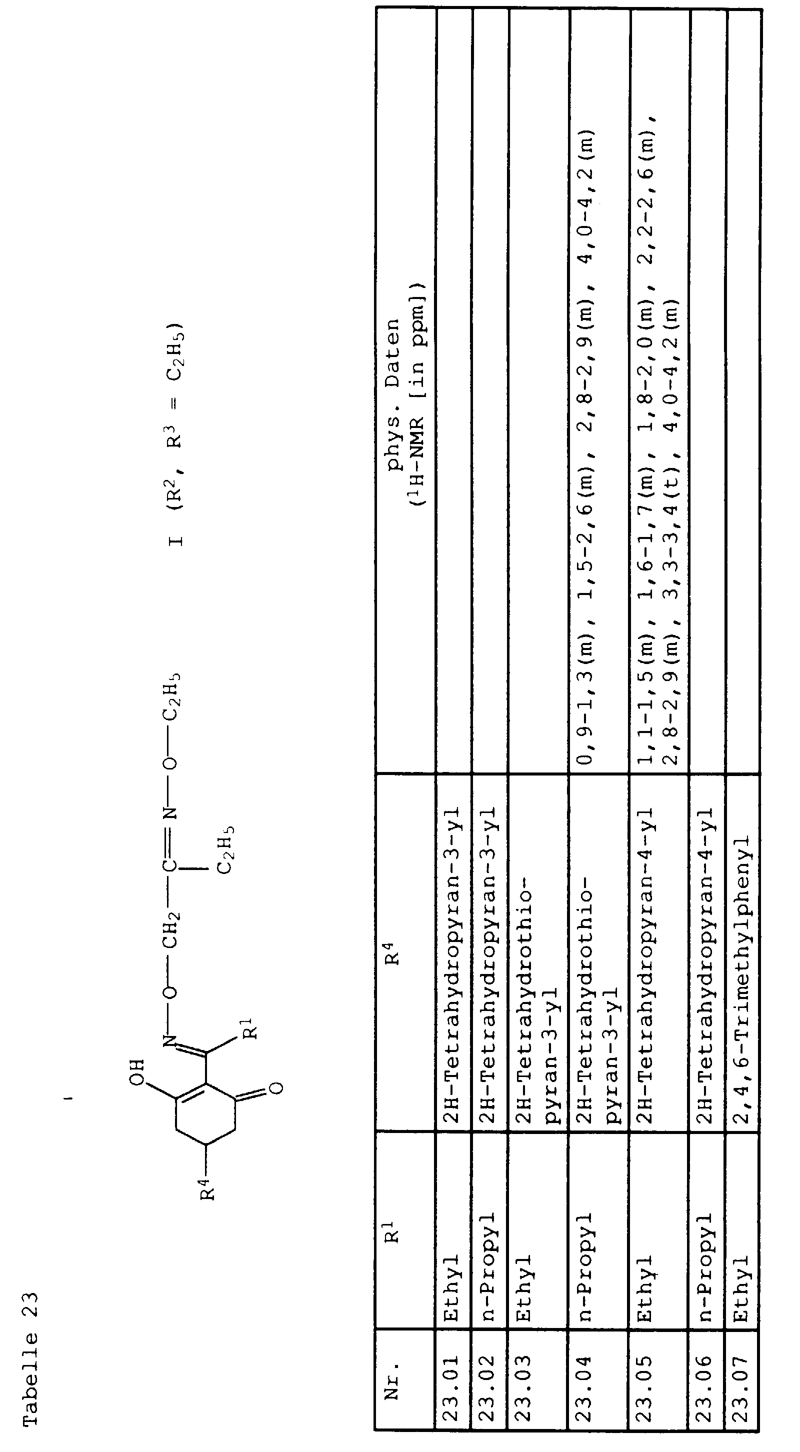

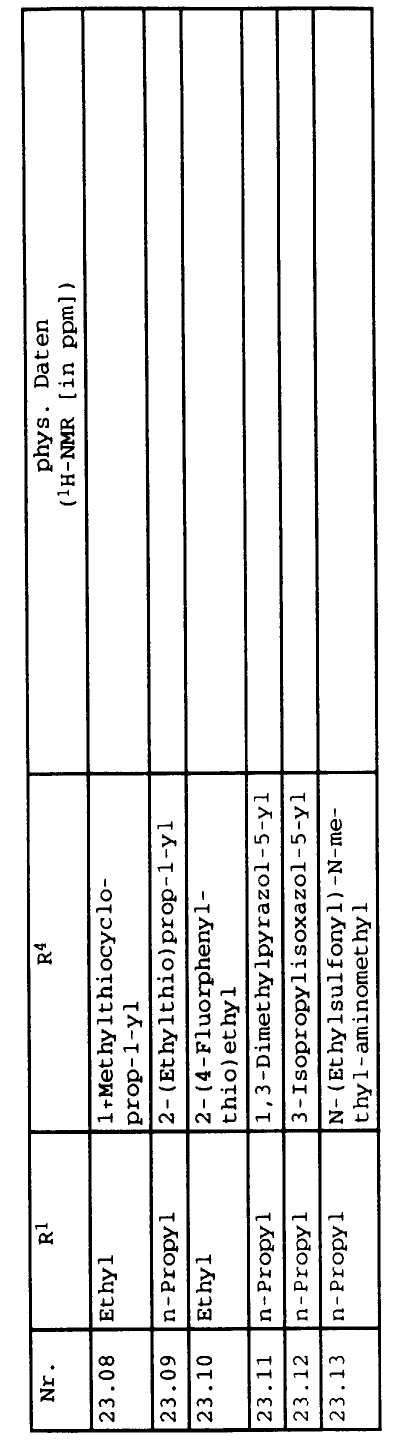

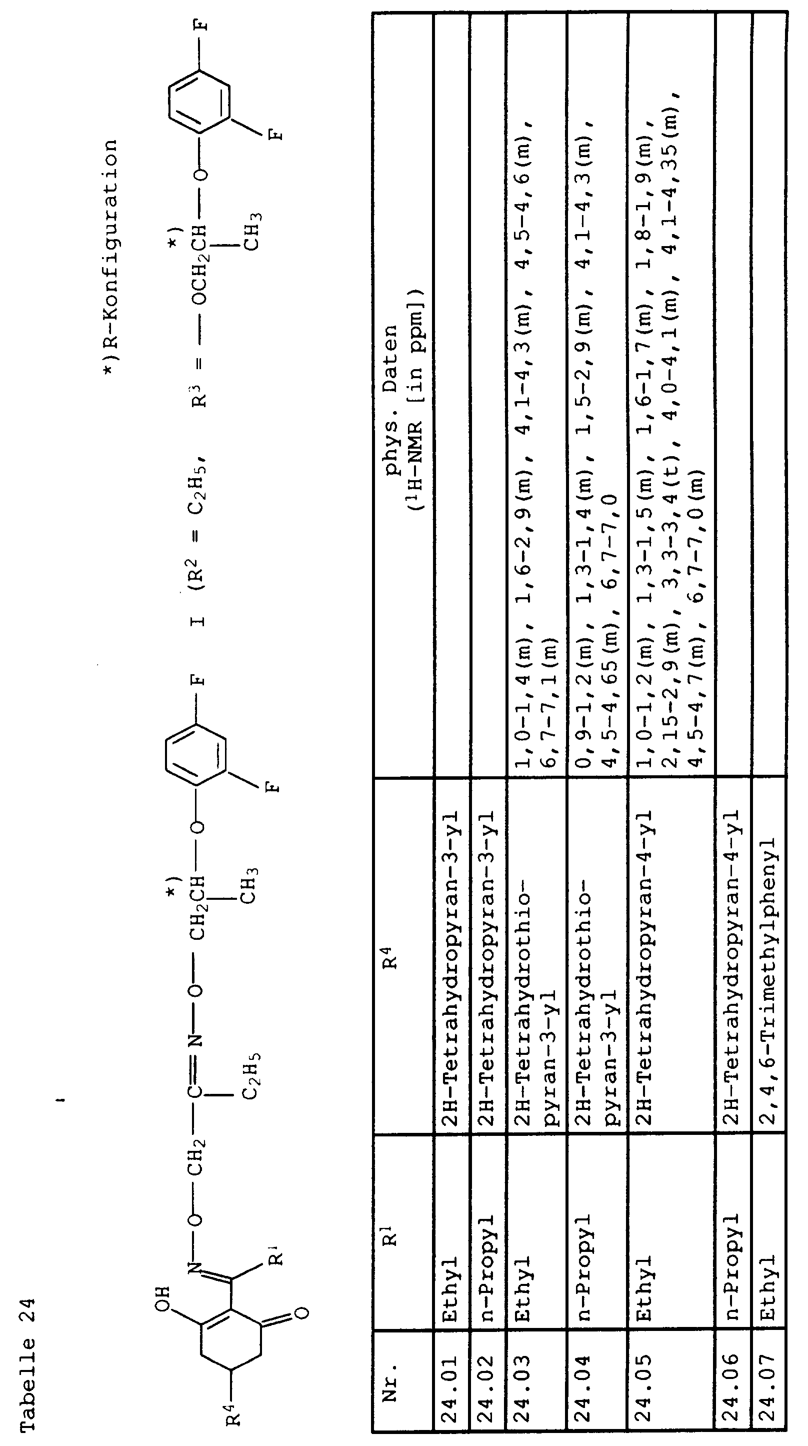

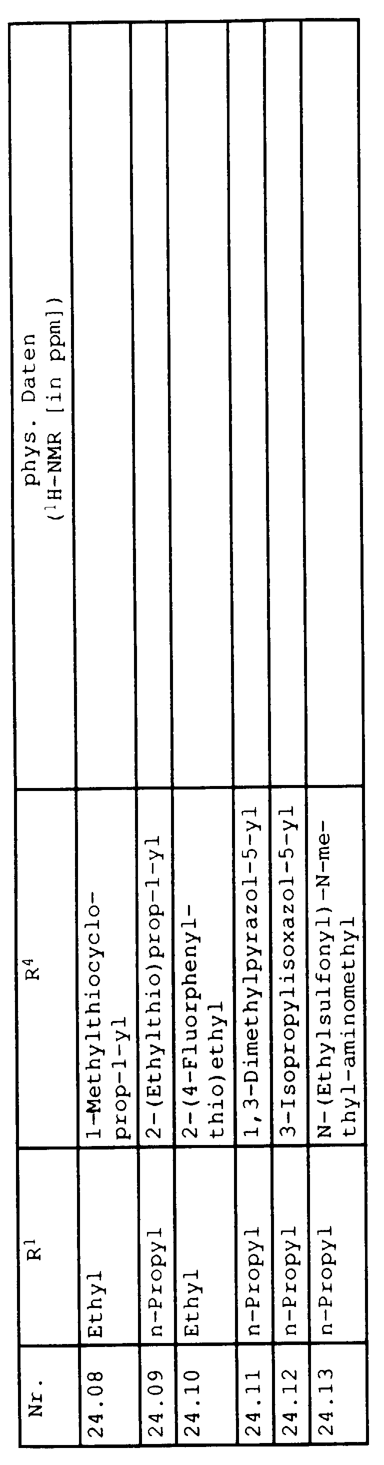

### Anwendungsbeispiele

Die herbizide Wirkung der O-(Oxyimino)ethyl-Cyclohexenonoximether der Formel I ließ sich durch Gewächshausversuche zeigen:

Als Kulturgefäße dienten Plastikblumentöpfe mit lehmigem Sand mit etwa 3,0% Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkte ein gleichmäßiges Keimen der Testpflanzen, sofern dies nicht durch die Wirkstoffe beeinträchtigt wurde.

Zum Zweck der Nachauflaufbehandlung werden die Testpflanzen je nach Wuchsform erst bis zu einer Wuchshöhe 3 bis 15cm angezogen und erst dann mit den in Wasser suspendierten oder emulgierten Wirkstoffen behandelt. Die Testpflanzen werden dafür entweder direkt gesät und in den gleichen Gefäßen aufgezogen oder sie werden erst als Keimpflanzen getrennt angezogen und einige Tage vor der Behandlung in die Versuchsgefäße verpflanzt. Die Aufwandmenge für die Nachauflaufbehandlung betrug 0,25 und 0,125 kg/ha an aktiver Substanz (a.S.).

Die Pflanzen wurden artenspezifisch bei Temperaturen von 10 -25 °C bzw. 20 - 35 °C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Echinochloa crus-gali | Hühnerhirse | barnyardgras |
| Setaria italica | Kolbenhirse | foxtail millet |
| Setaria faberii | Borstenhirse | giant foxtail |
| Setaria viridis | Grüne Borstenhirse | green foxtail |
| Orysa sativa | Reis | rice |
| Triticum aestivum | Winterweizen | winter wheat |

Das Ergebnis zeigte, daß mit den Verbindungen Nr. 3.05, 4.01, 4.03 und 9.04 unerwünschte Gräser in Weizen oder Reis als Beispielkulturen sehr gut bekämpft werden können.

## Patentansprüche

1. O-(Oxyimino)ethyl-Cyclohexenonoximether der Formel I 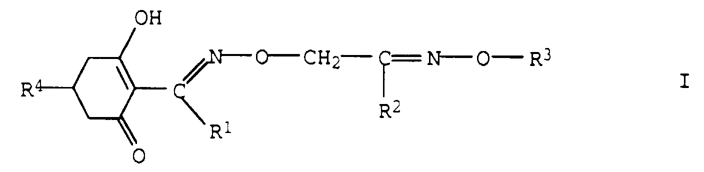 in der die Substituenten folgende Bedeutung haben:
R¹ eine C₁-C₆-Alkylgruppe;
R² eine C₁-C₆-Alkylgruppe;
R³ die Phenylgruppe, die unsubstituiert sein oder ein bis drei Substituenten tragen kann, ausgewählt aus der Gruppe bestehend aus Nitro, Cyano, Halogen, C₁-C₄-Alkyl und C₁-C₄-Halogenalkyl;
eine C₁-C₄-Alkyl-, C₃-C₄-Alkenyl- oder C₃-C₄-Alkinylgruppe, wobei diese Gruppen gewünschtenfalls einen der folgenden Substituenten tragen können:
Halogen, C₁-C₃-Alkyl, Phenyl, das gewünschtenfalls seinerseits ein bis drei Reste tragen kann, ausgewählt aus der Gruppe bestehend aus Nitro, Cyano, Halogen, C₁-C₄-Alkyl, C₁-C₄-Halogenalkyl, Phenyl und Phenoxy, oder
Phenoxy, das gewünschtenfalls seinerseits ein bis drei Reste tragen kann, ausgewählt aus der Gruppe bestehend aus Nitro, Cyano, Halogen, C₁-C₄-Alkyl und C₁-C₄-Halogenalkyl;
R⁴ eine C₁-C₄-Alkoxy-C₁-C₆-alkyl- oder C₁-C₄-Alkylthio-C₁-C₆-alkylgruppe;
eine Phenylthio-C₁-C₆-alkylgruppe, wobei der Phenylring gewünschtenfalls ein bis drei Substituenten tragen kann, ausgewählt aus der Gruppe bestehend aus Halogen und C₁-C₄-Halogenalkyl;
eine N-(C₁-C₄-Alkylsulfonyl)-N-(C₁-C₄-alkyl)aminomethylgruppe;
eine C₃-C₇-Cycloalkyl- oder C₅-C₇-Cycloalkenylgruppe, wobei diese Gruppen unsubstituiert sein oder jeweils ein bis drei Substituenten tragen können, ausgewählt aus der Gruppe bestehend aus Hydroxyl, Halogen, C₁-C₄-Alkyl, C₁-C₄-Alkoxy, C₁-C₄-Alkylthio, C₁-C₄-Halogenalkyl;
ein 5-gliedriger gesättigter Heterocyclus, der ein oder zwei Sauerstoff- und/oder Schwefelatome als Heteroatome enthält und der unsubstituiert sein oder ein bis drei Substituenten tragen kann, ausgewählt aus der Gruppe bestehend aus C₁-C₄-Alkyl, C₁-C₄-Alkoxy, C₁-C₄-Alkylthio und C₁-C₄-Halogenalkyl;
ein 6- oder 7-gliedriger Heterocyclus mit einem oder zwei nicht benachbarten Sauerstoff- und/oder Schwefelatomen als Heteroatomen, der gesättigt oder ein- oder zweifach ungesättigt sein kann, wobei der Heterocyclus unsubstituiert sein oder ein bis drei Substituenten tragen kann, ausgewählt aus der Gruppe bestehend aus Hydroxyl, Halogen, C₁-C₄-Alkyl, C₁-C₄-Alkoxy, C₁-C₄-Alkylthio und C₁-C₄-Halogenalkyl;
ein 5-gliedriger Heteroaromat, enthaltend ein oder zwei Stickstoffatome und ein Sauerstoff- oder Schwefelatom, wobei der Heteroaromat unsubstituiert sein oder ein bis drei Substituenten tragen kann, ausgewählt aus der Gruppe bestehend aus Halogen, Cyano, C₁-C₄-Alkyl, C₁-C₄-Alkoxy, C₁-C₄-Alkylthio, C₁-C₄-Halogenalkyl, C₂-C₆-Alkenyl, C₂-C₆-Alkenyloxy und C₁-C₄-Alkoxy-C₁-C₄-alkyl;
die Phenyl- oder Pyridylgruppe, wobei diese Gruppen unsubstituiert sein oder jeweils ein bis drei Substituenten tragen können, ausgewählt aus der Gruppe bestehend aus Halogen, Nitro, Cyano, C₁-C₄-Alkyl, C₁-C₄-Alkoxy, C₁-C₄-Alkylthio, C₁-C₄-Halogenalkyl, C₃-C₆-Alkenyloxy, C₃-C₆-Alkinyloxy und -NR^{a}R^{b},
wobei
R^{a} für Wasserstoff, C₁-C₄-Alkyl, C₃-C₆-Alkenyl oder C₃-C₆-Alkinyl und
R^{b} für Wasserstoff, C₁-C₄-Alkyl, C₃-C₆-Alkenyl, C₃-C₆-Alkinyl, C₁-C₆-Acyl oder Benzoyl, das gewünschtenfalls seinerseits ein bis drei Reste tragen kann, ausgewählt aus der Gruppe besehend aus Nitro, Cyano, Halogen, C₁-C₄-Alkyl, C₁-C₄-Alkoxy, C₁-C₄-Alkylthio und C₁-C₄-Halogenalkyl
stehen;
sowie die landwirtschaftlich brauchbaren Salze von I und die Ester von I mit C₁-C₁₀-Carbonsäuren oder anorganischen Säuren.

2. Verwendung der O-(Oxyimino)ethyl-Cyclohexenonoximether I und/oder deren landwirtschaftlich brauchbaren Salze und/oder Ester mit C₁-C₁₀-Carbonsäuren oder anorganischen Säuren, gemäß Anspruch 1, als Herbizide.

3. Herbizides Mittel, enthaltend eine herbizid wirksame Menge mindestens eines O-(Oxyimino)ethyl-Cyclohexenonoximethers der Formel I und/oder eines landwirtschaftlich brauchbaren Salzes von I und/oder Esters von I mit C₁-C₁₀-Carbonsäuren oder anorganischen Säuren, gemäß Anspruch 1, und mindestens einen flüssigen und/oder festen Trägerstoff und gewünschtenfalls mindestens ein Adjuvant.

4. Verfahren zur Herstellung von herbizid wirksamen Mitteln, dadurch gekennzeichnet, daß man eine herbizid wirksame Menge eines O-(Oxyimino)ethyl-Cyclohexenonoximethers der Formel I und/oder eines landwirtschaftlich brauchbaren Salzes von I und/oder Esters von I mit C₁-C₁₀-Carbonsäuren oder anorganischen Säuren, gemäß Anspruch 1, mit mindestens einem flüssigen und/oder festen Trägerstoff und gewünschtenfalls mit mindestens einem Adjuvant mischt.

5. Verfahren zur Bekämpfung von unerwünschtem Pflanzenwuchs, dadurch gekennzeichnet, daß man eine herbizid wirksame Menge mindestens eines O-(Oxyimino)ethyl-Cyclohexenonoximethers der Formel I und/oder eines landwirtschaftlich brauchbaren Salzes von I und/oder Esters von I mit C₁-C₁₀-Carbonsäuren oder anorganischen Säuren, gemäß Anspruch 1, auf Pflanzen, deren Lebensraum oder auf Saatgut einwirken läßt.

6. O-(Oxyimino)ethyl-Hydroxylamine der Formel III 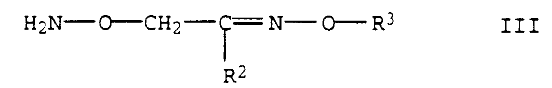 wobei
R² eine C₁-C₆-Alkylgruppe und
R³ die Phenylgruppe, die unsubstituiert sein oder ein bis drei Substituenten tragen kann, ausgewählt aus der Gruppe bestehend aus Nitro, Cyano, Halogen, C₁-C₄-Alkyl und C₁-C₄-Halogenalkyl oder
eine C₁-C₄-Alkyl-, C₃-C₄-Alkenyl- oder C₃-C₄-Alkinylgruppe, wobei diese Gruppen gewünschtenfalls einen der folgenden Substituenten tragen können:
Halogen, C₁-C₃-Alkyl,
Phenyl, das unsubstituiert sein oder seinerseits ein bis drei Reste tragen kann, ausgewählt aus der Gruppe bestehend aus Nitro, Cyano, Halogen, C₁-C₄-Alkyl, C₁-C₄-Halogenalkyl, Phenyl und Phenoxy, oder
Phenoxy, das gewünschtenfalls seinerseits ein bis drei Reste tragen kann, ausgewählt aus der Gruppe bestehend aus Nitro, Cyano, Halogen, C₁-C₄-Alkyl und C₁-C₄-Halogenalkyl
bedeuten,
sowie die Ammonium-Salze der Verbindungen III mit Mineralsäuren.

## Claims

1. An O-(oximino)ethylcyclohexenone oxime ether of the formula I 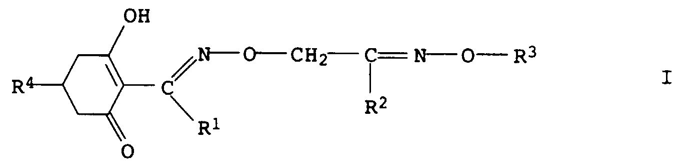 where the substituents have the following meanings:
R¹ is a C₁-C₆-alkyl group;
R² is a C₁-C₆-alkyl group;
R³ is the phenyl group, which can be unsubstituted or can carry one to three substituents selected from the group consisting of nitro, cyano, halogen, C₁-C₄-alkyl and C₁-C₄-haloalkyl;
a C₁-C₄-alkyl, C₃-C₄-alkenyl or C₃-C₄-alkynyl group, these groups, if desired, being able to carry one of the following substituents:
halogen, C₁-C₃-alkyl, phenyl which, if desired, in turn can carry one to three radicals selected from the group consisting of nitro, cyano, halogen, C₁-C₄-alkyl, C₁-C₄-haloalkyl, phenyl and phenoxy, or phenoxy which, if desired, in turn can carry one to three radicals selected from the group consisting of nitro, cyano, halogen, C₁-C₄-alkyl and C₁-C₄-haloalkyl;
R⁴ is a C₁-C₄-alkoxy-C₁-C₆-alkyl or C₁-C₄-alkylthio-C₁-C₆-alkyl group;
a phenylthio-C₁-C₆-alkyl group, the phenyl ring, if desired, being able to carry one to three substituents selected from the group consisting of halogen and C₁-C₄-haloalkyl;
an N-(C₁-C₄-alkylsulfonyl)-N-(C₁-C₄-alkyl)aminomethyl group;
a C₃-C₇-cycloalkyl or C₅-C₇-cycloalkenyl group, where these groups can be unsubstituted or in each case can carry one to three substituents selected from the group consisting of hydroxyl, halogen, C₁-C₄-alkyl, C₁-C₄-alkoxy, C₁-C₄-alkylthio and C₁-C₄-haloalkyl;
a 5-membered saturated heterocycle which contains one or two oxygen and/or sulfur atoms as heteroatoms and which can be unsubstituted or can carry one to three substituents selected from the group consisting of C₁-C₄-alkyl, C₁-C₄-alkoxy, C₁-C₄-alkylthio and C₁-C₄-haloalkyl;
a 6- or 7-membered heterocycle having one or two non-adjacent oxygen and/or sulfur atoms as heteroatoms, which can be saturated or mono- or diunsaturated, where the heterocycle can be unsubstituted or can carry one to three substituents selected from the group consisting of hydroxyl, halogen, C₁-C₄-alkyl, C₁-C₄-alkoxy, C₁-C₄-alkylthio and C₁-C₄-haloalkyl;
a 5-membered heteroaromatic containing one or two nitrogen atoms and an oxygen or sulfur atom, where the heteroaromatic can be unsubstituted or can carry one to three substituents selected from the group consisting of halogen, cyano, C₁-C₄-alkyl, C₁-C₄-alkoxy, C₁-C₄-alkylthio, C₁-C₄-haloalkyl, C₂-C₆-alkenyl, C₂-C₆-alkenyloxy and C₁-C₄-alkoxy-C₁-C₄-alkyl;
the phenyl or pyridyl group, where these groups can be unsubstituted or in each case can carry one to three substituents selected from the group consisting of halogen, nitro, cyano, C₁-C₄-alkyl, C₁-C₄-alkoxy, C₁-C₄-alkylthio, C₁-C₄-haloalkyl, C₃-C₆-alkenyloxy, C₃-C₆-alkynyloxy and -NR^{a}R^{b},
where
R^{a} is hydrogen, C₁-C₄-alkyl, C₃-C₆-alkenyl or C₃-C₆-alkynyl and
R^{b} is hydrogen, C₁-C₄-alkyl, C₃-C₆-alkenyl, C₃-C₆-alkynyl, C₁-C₆-acyl or benzoyl which, if desired, in turn can carry one to three radicals selected from the group consisting of nitro, cyano, halogen, C₁-C₄-alkyl, C₁-C₄-alkoxy, C₁-C₄-alkylthio and C₁-C₄-haloalkyl;
and the agriculturally utilizable salts of I and the esters of I with C₁-C₁₀-carboxylic acids or inorganic acids.

2. The use of the O-(oximino)ethylcyclohexenone oxime ethers I and/or their agriculturally utilizable salts and/or esters with C₁-C₁₀-carboxylic acids or inorganic acids, as claimed in claim 1, as herbicides.

3. A herbicidal composition containing a herbicidally effective amount of at least one O-(oximino)ethylcyclohexenone oxime ether of the formula I and/or of an agriculturally utilizable salt of I and/or ester of I with C₁-C₁₀-carboxylic acids or inorganic acids, as claimed in claim 1, and at least one liquid and/or solid carrier and, if desired, at least one adjuvant.

4. A process for the production of herbicidally effective compositions, which comprises mixing a herbicidally effective amount of an O-(oximino)ethylcyclohexenone oxime ether of the formula I and/or of an agriculturally utilizable salt of I and/or ester of I with C₁-C₁₀-carboxylic acids or inorganic acids, as claimed in claim 1, with at least one liquid and/or solid carrier and, if desired, with at least one adjuvant.

5. A process for controlling undesired plant growth, which comprises allowing a herbicidally effective amount of at least one O-(oximino)ethylcyclohexenone oxime ether of the formula I and/or of an agriculturally utilizable salt of I and/or ester of I with C₁-C₁₀-carboxylic acids or inorganic acids, as claimed in claim 1, to act on plants, their environment or on seeds.

6. An O-(oximino)ethylhydroxylamine of the formula III 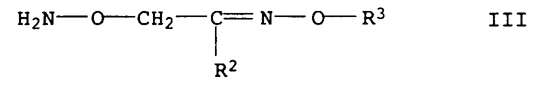 where
R² is a C₁-C₆-alkyl group and
R³ is the phenyl group, which can be unsubstituted or can carry one to three substituents selected from the group consisting of nitro, cyano, halogen, C₁-C₄-alkyl and C₁-C₄-haloalkyl or
a C₁-C₄-alkyl, C₃-C₄-alkenyl or C₃-C₄-alkynyl group, where these groups, if desired, can carry one of the following substituents:
halogen, C₁-C₃-alkyl,
phenyl which can be unsubstituted or in turn can carry one to three radicals selected from the group consisting of nitro, cyano, halogen, C₁-C₄-alkyl, C₁-C₄-haloalkyl, phenyl and phenoxy, or
phenoxy which, if desired, in turn can carry one to three radicals selected from the group consisting of nitro, cyano, halogen, C₁-C₄-alkyl and C₁-C₄-haloalkyl,
and the ammonium salts of the compounds III with inorganic acids.

## Revendications

1. Ethers d'O-(oxyimino)éthyl-cyclohexénonoximes de formule I 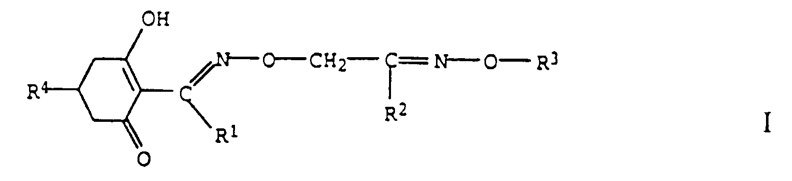 dans laquelle les symboles ont les significations suivantes :
R¹ : un groupe alkyle en C1-C6;
R² : un groupe alkyle en C1-C6;
R³ : le groupe phényle non substitué ou portant un à trois substituants choisis parmi les groupes nitro, cyano, les halogènes, les groupes alkyle en C1-C4 et halogénoalkyle en C1-C4;
un groupe alkyle en C1-C4, alcényle en C3-C4 ou alcynyle en C3-C4, qui peuvent, si on le désire, porter un des substituants suivants : un halogène, un groupe alkyle en C1-C3, phényle lequel peut, si on le désire, porter un à trois substituants choisis parmi les groupes nitro, cyano, les halogènes, les groupes alkyle en C1-C4, halogénoalkyle en C1-C4, phényle et phénoxy, ou bien
un groupe phénoxy qui peut lui-même porter, si on le désire, un à trois substituants choisis parmi les groupes nitro, cyano, les halogènes, les groupes alkyle en C1-C4 et halognéoalkyle en C1-C4 ;
R⁴ : un groupe (alcoxy en C1-C4)alkyle en C1-C6 ou (alkyle en C1-C4)-thioalkyle en C1-C6;
un groupe phénylthio-alkyle en C1-C6, le cycle phényle pouvant, si on le désire, porter un à trois substituants choisis parmi les halogènes et les groupes halogénoalkyle en C1-C4; un groupe N-(aclkylsulfonyle en C1-C4)-N-(alkyle en C1-C4)-aminométhyle;
un groupe cycloalkyle en C3-C7 ou cycloalcényle en C5-C7, ces groupes pouvant être non substitués ou porter chacun un à trois substituants choisis parmi les groupes hydroxy, les halogènes, les groupes alkyle en C1-C4, alcoxy en C1-C4, alkylthio en C1-C4, halogénoalkyle en C1-C4;
un hétérocycle saturé à cinq chaînons qui contient, en tant qu'hétéroatomes, deux atomes d'oxygène et/ou de soufre et qui est non substitué ou peut porter un à trois substituants choisis parmi les groupes alkyle en C1-C4, alcoxy en C1-C4, alkylthio en C1-C4 et halogénoalkyle en C1-C4;
un hétérocycle à six ou sept chaînons contenant, en tant qu'hétéroatomes, un ou deux atomes d'oxygène et/ou de soufre non voisins, qui peut être saturé ou mono- ou di- insaturé, l'hétérocycle pouvant être non substitué ou pouvant porter un à trois substituants choisis parmi les groupes hydroxy, les halogènes, les groupes alkyle en C1-C4, alcoxy en C1-C4, alkylthio en C1-C4 et halogénoalkyle en C1-C4;
un radical hétéroaromatique à cinq chaînons contenant un ou deux atomes d'azote et un atome d'oxygène ou de soufre, le radical hétéroaromatique étant non substitué ou portant un à trois substitutants choisis parmi les halogènes, les groupes cyano, alkyle en C1-C4, alcoxy en C1-C4, alkylthio en C1-C4, halogénoalkyle en C1-C4, alcényle en C2-C6, alcényloxy en C2-C6 et (alcoxy en C1-C4)alkyle en C1-C4;
un groupe phényle ou pyridyle, ces groupes pouvant être non substitués ou porter chacun un à trois substituants choisis parmi les halogènes, les groupes nitro, cyano, alkyle en C1-C4, alcoxy en C1-C4, alkylthio en C1-C4, halogénoalkyle en C1-C4, alcényloxy en C3-C6, alcynyloxy en C3-C6 et -NR^{a}R^{b}.
dans lequel
R^{a} représente l'hydrogène, un groupe alkyle en C1-C4, alcényle en C3-C6 ou alcynyle en C3-C6 et
R^{b} représente l'hydrogène, un groupe alkyle en C1-C4, alcényle en C3-C6, alcynyle en C3-C6, acyle en C1-C6 ou benzoyle qui peut lui-même porter un à trois substituants choisis parmi les groupes nitro, cyano, les halogènes, les groupes alkyle en C1-C4, alcoxy en C1-C4, alkylthio en C1-C4 et halogénoalkyle en C1-C4;
ainsi que les sels des composés I et les esters des composés I et d'acides carboxyliques en C1-C10 ou d'acides minéraux convenant pour les applications agricoles.

2. Utilisation des éthers d'O-(oxyimino)éthyl-cyclohexénonoximes I et/ou de leurs sels et/ou esters d'acides carboxyliques en C1-C10 ou d'acides minéraux convenant pour les applications agricoles, selon la revendication 1, en tant qu'herbicides.

3. Produit herbicide contenant une quantité herbicide efficace d'au moins un éther d'O-(oxyimino)éthyl-cyclohexénonoxime de formule I et/ou d'un sel d'un composé I et/ou d'un ester d'un composé I et d'un acide carboxylique en C1-C10 ou d'un acide minéral, acceptable pour les applications agricoles, selon la revendication 1, et au moins un véhicule liquide et/ou solide avec, si on le désire, au moins un adjuvant.

4. Procédé pour préparer des produits à activité herbicides, caractérisé par le fait que l'on mélange une quantité herbicide efficace d'un éther d'O-(oxyimino)éthyl-cyclohexénonoxime de formule I et/ou d'un sel d'un composé I et/ou d'un ester d'un composé I et d'un acide carboxylique en C1-C10 ou d'un acide minéral, acceptable pour les applications agricoles, selon la revendication 1, avec au moins un véhicule liquide et/ou solide et, si on le désire, au moins un adjuvant.

5. Procédé pour combattre les croissances de végétaux indésirables, caractérisé par le fait que l'on fait agir sur les végétaux, leur habitat ou leurs semences, une quantité herbicide efficace d'au moins un éther d'O-(oxyimino)éthyl-cyclohexénonoxime de formule I et/ou d'un sel d'un composé I et/ou d'un ester d'un composé I et d'un acide carboxylique en C1-C10 ou d'un acide minéral, acceptable pour les applications agricoles selon la revendication 1.

6. O-(oxyimino)éthyl-hydroxylamines de formule III 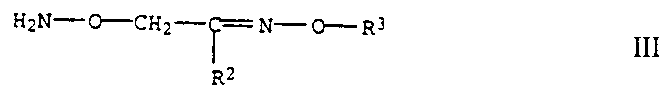 dans laquelle
R² représente un groupe alkyle en C1-C6 et
R³ représente le groupe phényle non substitué ou portant un à trois substituants choisis parmi les groupes nitro, cyano, les halogènes, les groupes alkyle en C1-C4 et halogénoalkyle en C1-C4, ou bien un groupe alkyle en C1-C4, alcényle en C3-C4 ou alcynyle en C3-C4, ces groupes pouvant, si on le désire, porter l'un des substituants suivants :
un halogène, un groupe alkyle en C1-C3,
un groupe phényle non substitué ou portant un à trois substituants choisis parmi les groupes nitro, cyano, les halogènes, les groupes alkyle en C1-C4, halogénoalkyle en C1-C4, phényle et phénoxy,
ou un groupe phénoxy qui peut porter un à trois substituants choisis parmi les groupes nitro, cyano, les halogènes, les groupes alkyle en C1-C4 et halogénoalkyle en C1-C4,
ainsi que les sels d'ammonium des composés III et d'acides minéraux.
